# EUROPEAN PATENT SPECIFICATION

(11) **EP 4 495 125 B1**
(45) Date of publication and mention of the grant of the patent: **09.09.2026**
(21) Application number: 24189423.7
(22) Date of filing: 18.07.2024
(51) Int. Cl.: C07F 7/18, C07C 41/01, C07B 41/04, C07C 41/26, C07C 45/51, C07C 253/30, C07C 403/16, C07J 5/00

(54) **A CATALYST FREE SYNTHESIS METHOD FOR INTRODUCING A TRIFLUOROMETHOXY MOIETY INTO AT LEAST ONE ORGANIC COMPOUND**
KATALYSATORFREIES SYNTHESEVERFAHREN ZUR EINFÜHRUNG EINER TRIFLUORMETHOXYEINHEIT IN MINDESTENS EINE ORGANISCHE VERBINDUNG
PROCÉDÉ DE SYNTHÈSE SANS CATALYSEUR POUR INTRODUIRE UN FRAGMENT TRIFLUOROMÉTHOXY DANS AU MOINS UN COMPOSÉ ORGANIQUE

(30) Priority: 19.07.2023 EP 23186390; 16.11.2023 EP 23210358
(43) Date of publication of application: 22.01.2025
(73) Proprietor: Freie Universität Berlin, 14195 Berlin (DE)
(72) Inventor: HASENSTAB-RIEDEL, Sebastian, 14532 Kleinmachnow (DE); HOPKINSON, Matthew, Tyne and Wear, Ne270FL (GB); MAAS, Lilian Maria, 14167 Berlin (DE)
(74) Representative: Maikowski & Ninnemann Patentanwälte Partnerschaft mbB

(56) References cited:
- EP-A1- 2 628 722
- WO-A1-2012/087519
- US-A- 4 296 236
- ZRIBA RIADH ET AL: "Preparation and Reactivity of some New Keto- and Styrene-Based Trifluoromethoxylated Synthons", vol. 2009, no. 07, 1 April 2009 (2009-04-01), DE, pages 1131 - 1135, XP093108216, ISSN: 0936-5214, Retrieved from the Internet <URL:https://www.thieme-connect.de/products/ejournals/pdf/10.1055/s-0028-1088155.pdf> DOI: 10.1055/s-0028-1088155
- DATABASE REAXYS [online] 1 January 2013 (2013-01-01), CENTRE NATIONAL DE LA RECHERCHE SCIENTIFIQUE; BAYER AG: "CF3O-containing enaminoketones and their utilization for the preparation of CF3O-containing pyrazoles", XP093108217, Database accession no. EP2628722 A1
- DATABASE REAXYS [online] 1 January 2012 (2012-01-01), NOVARTIS AG: "A method for adjusting the composition and FXR", XP093108218, Database accession no. WO2012/87519 A1
- MARZI E ET AL: "The site-selective functionalization of halogen-bearing phenols: an exercise in diversity-oriented organometallic synthesis", TETRAHEDRON, ELSEVIER SIENCE PUBLISHERS, AMSTERDAM, NL, vol. 61, no. 13, 28 March 2005 (2005-03-28), pages 3393 - 3401, XP027861007, ISSN: 0040-4020, [retrieved on 20050328]
- DATABASE REAXYS [online] 1 January 1981 (1981-01-01), MERCK & CO INC: "US4296236", XP093108219, Database accession no. Rx-ID: 25126323

## Description

The present invention relates to a catalyst free synthesis method for introducing a trifluoromethoxy moiety into at least one organic compound.

The trifluoromethoxy group and its introduction into organic molecules is of growing interest in several different fields, especially agro- and medicinal chemistry, due to the group's unique properties and good metabolic stability. Often referred to as a "super halogen", the group exhibits high lipophilicity (π = 1.04) as well as comparable electronegativity to a single fluorine atom (χ^{OGF3} = 3.7, χ^{F} = 3.9) and a lower electron-withdrawing effect compared to other fluorinated groups. These properties make the OCF₃ group ideal for fine-tuning the chemico-biological activity of drug and agrochemical targets (A. Tlili, F. Toulgoat, T. Billard, Angew. Chem. Int. Ed. 2016, 55, 11726-11735.; B.-Y. Hao, Y.-P. Han, Y. Zhang, Y.-M. Liang, Organic & Biomolecular Chemistry 2023, 21, 4926-4954).

Despite this advantage, only a few compounds containing an OCF₃ moiety are currently used and, only 1.5% of fluorine bearing pharmaceuticals and 2.5% of fluoro-agrochemicals on the market are trifluoromethoxylated (J. Liu, W. Lin, A. E. Sorochinsky, G. Butler, A. Landa, J. Han, V. A. Soloshonok, J Fluorine Chem 2022, 257-258, 109978). This might result from the lack of practical methods for introducing OCF₃, especially in the late stage of a synthetic route. Known methods for the direct introduction of OCF₃ can be separated into nucleophilic and radical approaches. Most commonly nucleophilic transformations have been employed; however, these are inherently challenging due to the instability of ⁻OCF₃ towards *β*-fluoride elimination. Radical trifluoromethoxylation has been traditionally seldom conducted, however, in recent years the development of new radical OCF₃-transfer reagents has reinvigorated the field, opening up new synthetic routes towards trifluoromethoxylated molecules.

In 2018, the groups of Ngai and Togni, followed by Tang and co-workers in 2020, introduced bench stable reagents which release OCF₃ radicals upon photocatalytic activation of an N-O or S-O bond (see Scheme 1) (W. Zheng, C. A. Morales-Rivera, J. W. Lee, P. Liu, M.-Y. Ngai, Angew. Chem. Int. Ed. 2018, 57, 9645-9649; W. Zheng, J. W. Lee, C. A. Morales-Rivera, P. Liu, M.-Y. Ngai, Angew. Chem. Int. Ed. 2018, 57, 13795-13799.; B. J. Jelier, P. F. Tripet, E. Pietrasiak, I. Franzoni, G. Jeschke, A. Togni, Angew. Chem. Int. Ed. 2018, 57, 13784-13789; Z. Deng, M. Zhao, F. Wang, P. Tang, Nature Communications 2020, 11, 2569).

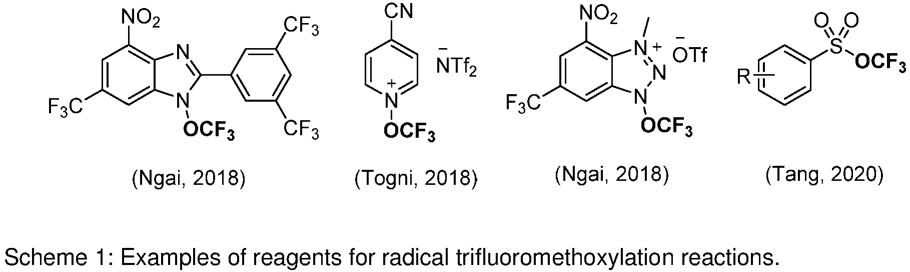

Given the expensive synthesis and low atom economy of these reagents, Dix et al. (S. Dix, P. Golz, J. R. Schmid, S. Riedel, M. N. Hopkinson, Chemistry 2021, 27, 11554-11558) recently developed bis(trifluoromethyl)-peroxide (BTMP) as a practical and comparatively inexpensive reagent for the direct trifluoromethoxylation of (hetero)arenes (Scheme 2).

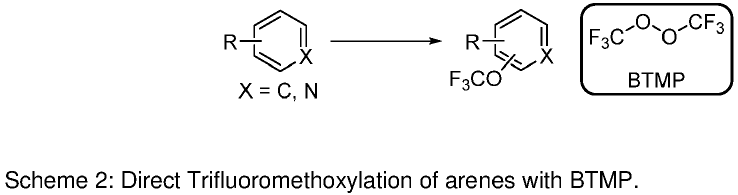

US 4 296 236 A describes in one Example the use of bis(trifluoromethyl)-peroxide (BTMP) for introducing a trifluoromethoxy moiety into Cephalosporanic acid ester.

Nevertheless, the number of methods for the aliphatic trifluoromethoxylation remains small. In 2021, Duhail et al. (T. Duhail, T. Bortolato, J. Mateos, E. Anselmi, B. Jelier, A. Togni, E. Magnier, G. Dagousset, L. Dell'Amico, Organic Letters 2021, 23, 7088-7093) presented the direct synthesis of α-trifluoromethoxylated ketones using the Togni OCF₃ reagent under photoredox catalytic activation.

It is thus desirable to provide alternative methods for introducing fluorinated alkoxy moieties into organic compounds. It is of a particular interest to find alternative methods for trifluoromethoxylation of organic compounds comprising silyl moieties.

Insofar as the term embodiment or aspect or alternative is used in the following, or features are presented as being optional, this should be interpreted in such a way that the only protection sought is that of the invention claimed and defined in the appended claims.

According the invention, a synthesis method, preferably a catalyst free synthesis method, for introducing a trifluoromethoxy moiety, into at least one organic compound has been provided, wherein the method comprises the following steps:
- Providing at least one organic compound comprising at least one silyl moiety as part of the following structural elements (I) or (II) 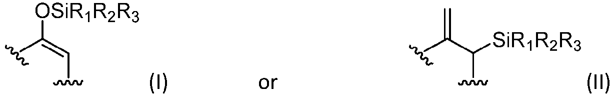 With R₁, R₂, R₃ being selected from C₁-C₄ Alkyl or C₆ Aryl, in particular -CH₃, -C₂H₅, -C₃H₇, -C₄H₉, -C₆H₅,
   Wherein R₁, R₂, R₃ can be the same or different,
- Reacting the at least one organic compound with the structural elements (I) or (II) with Bis(trifluoromethyl)-peroxide (BTMP) in the presence of at least one HF scavenger and at least one organic solvent, and
- Obtaining at least one organic compound comprising one of the following structural elements (III), (IV) or (IV) each with at least one trifluoromethoxy moiety

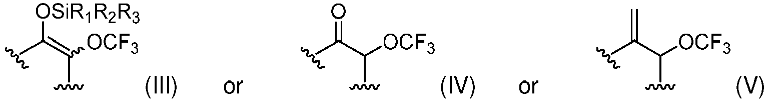

Thus, preferably a catalyst free method is provided that allows for trifluoromethoxylation of ketones in form of silyl enol ethers or of allyl silanes. Radical addition to the electron rich alkene in these compounds delivers trifluoromethoxylated silyl enol ethers, α-trifluoromethoxy ketones or trifluoromethoxylated allyl silanes.

It is to be understood that it is in general possible to use other peroxide compounds beside Bis(trifluoromethyl)-peroxide (BTMP). This is of particular interest if other fluorinated alkoxy groups besides -OCF₃ shall be introduced into any organic compound. Possible peroxides would contain for example (C₂F₅O)₂, (C₃F₇O)₂ (linear or branched) or (C₄F₉O)₂ (linear or branched).

In an embodiment of the present method, the at least silyl moiety is selected from -Si(CH₃)₃ (TMS, Trimethylsilyl), -Si(C₂H₅)₃ (TES, Triethylsilyl), -Si(CH₃)₂(C₄H₉) (TBDMS tertButyldimethylsilyl), -Si(C₃H₇)₃ (TIPS, Triisopropylsilyl), -Si(C₆H₅)₂(C₄H₉) (TBDPS, tert-Butyldiphenylsilyl). The mostly preferred silyl moiety is -Si(C₃H₇)₃ (TIPS, Triisopropylsilyl).

In a further embodiment of the present method the at least one HF scavenger is K₂CO₃, Na₂CO₃ or Cs₂CO₃, mostly preferred K₂CO₃.

In still another embodiment of the present method the at least one organic solvent is selected from a group containing dichloromethane (DCM), Acetone, acetonitrile (MeCN), Diethyl ether, Nitromethane, Tetrahydrofuran (THF), Dimethyl sulphoxide (DMSO) and Ethyl acetate.

The catalyst free reaction is carried out at a temperature between 18°C and 25°C, preferably between 20°C and 23°C, such as room temperature. Thus, the method can be carried out at ambient temperatures.

In a preferred embodiment, by reacting the organic compound with structural element (I) 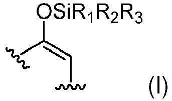 with Bis(trifluoromethyl)peroxide (BTMP) in the presence of at least one HF scavenger and at least one organic solvent an organic compound comprising one of the structural elements (III) or (IV) with at least trifluoromethoxy moiety. 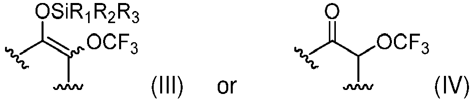 is provided.

The organic compound with structural element (I) used as starting material may be one of the following compounds:

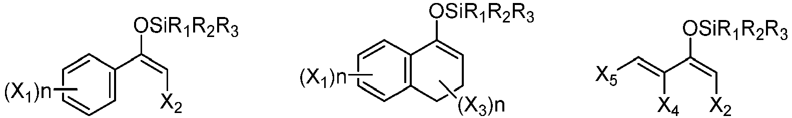

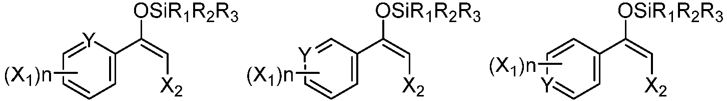

With
X₁ being H, alkyl, alkoxy, -CN, halogen, -OCH₂C₆H₅ (OBn), -CH₂C₆H₅ (Bn), -CₙF₂ₙ₊₁, an alkyl ring fused to the C₆ aryl;
X₂ being H, alkyl, aryl, heteroaryl, -CH₂C₆H₅ (Bn)
X₃ being H or alkyl,
X₄ being H or alkyl,
X₅ being alkyl, or cycloalkyl,
n being 0-5, and
Y being, N, S, or O.

In a preferred embodiment,
X₁ being H, C₁-C₆ alkyl, such as methyl, ethyl, propyl, butyl, C₁-C₆ alkoxy, -CN, F, Cl, Br, I, -OCH₂C₆H₅ (OBn), -CH₂C₆H₅ (Bn), -CF₃, -C₂F₅, or a C₅-C₆ alkyl ring, such as C₆ alkyl ring fused with the C₆ aryl;
X₂ being H, C₁-C₆ alkyl, C6 aryl, or -CH₂C₆H₅ (Bn)
X₃ being H or C₁-C₆ alkyl,
X₄ being H or C₁-C₆ alkyl,
X₅ being C₁-C₆ alkyl, such as C₆ alkyl, or C₃-C₆ cycloalkyl, such as C₆ cycloalkyl,
n being 0, 1, 2 or 3, and
Y being N, S, or O.

It is to be understood that any of the moieties mentioned above can be non-substituted or substituted.

Here the term "substituted", in particular in connection to alkyl, cycloalkyl, aryl relates to the substitution of one or more atoms, usually H-atoms, by one or more of the following substituents: halogen, hydroxy, protected hydroxy, oxo, C₁-C₁₅ alkyl, C₃-C₁₀-cycloalkyl, aryl, heteroaryl, naphthyl, imino, imido, isocyano, amino, protected amino, primary or secondary amino, heterocyclic ring, carbonate, imidazolyl, indolyl, pyrrolidinyl, C₁-C₁₂-alkoxy, C₁-C₁₂-acyl, C₁-C₁₂-acyloxy, nitro, nitroso, carboxy, ester, aldehyde, ketone, carbamoyl, carboxamide, N-(C₁-C₁₂-alkyl)carboxamide, C₁-C₁₂-(per)fluoroalkyl and C₁-C₁₀-alkylsulfonyl.

It is in preferred if any of the moieties mentioned above can be non-substituted or substituted with halogen, hydroxy, protected hydroxy, oxo, C₁-C₁₅ alkyl, C₃-C₁₀-cycloalkyl, aryl, heteroaryl, naphthyl, imino, imido, amino, protected amino, primary or secondary amino, C₁-C₁₂-alkoxy.

It is in particular preferred if any of the moieties mentioned above can be non-substituted or substituted with halogen, hydroxy, C₁-C₁₀ alkyl, such as methyl, ethyl or propyl, C₃-C₁₀-cycloalkyl, C₁-C₁₂-alkoxy.

The substituted groups can be once or twice substituted with same or different substituents. The alkyl moieties may also comprise one or multiple double bonds.

In preferred embodiments the organic compound with structural element (I) used as starting material may be one of the following compounds:

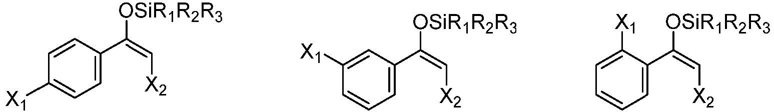

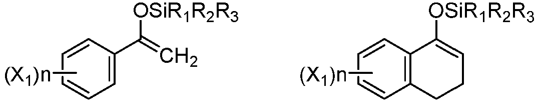

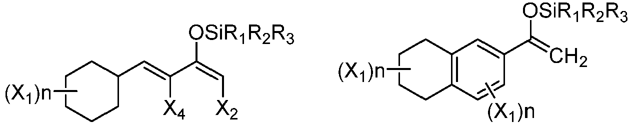

Surprisingly, the type of organic solvent used influences the reaction products obtained in the present method.

In one variant, when reacting the organic compound with structural element (I) as starting material 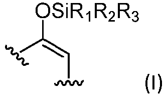 with Bis(trifluoromethyl)-peroxide (BTMP) in the presence of at least one HF scavenger and dichloromethane (DCM), diethyl ether and/or nitromethane, preferably dichloromethane (DCM), as the at least one organic solvent, an organic compound comprising the structural element (III) with at least one trifluoromethoxy moiety 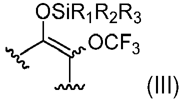 Is provided, i.e. trifluoromethoxylated silyl enol ethers are obtained.

When using dichloromethane (DCM), diethyl ether and/or nitromethane, preferably dichloromethane (DCM), as organic solvent, in an embodiment the following conversions may result:

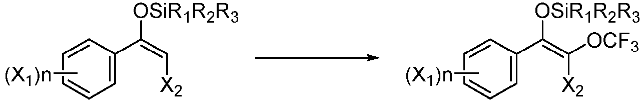

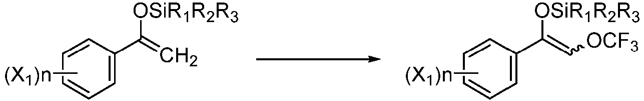

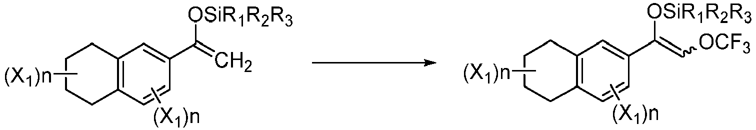

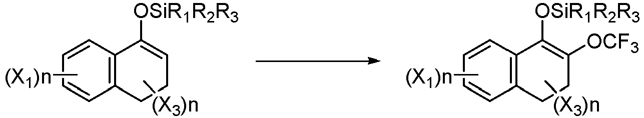

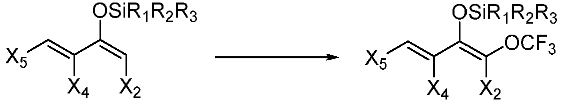

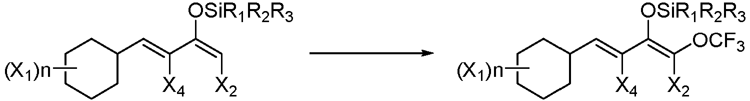

Wherein R₁, R₂, R₃, X₁, X₂, X₃, X₄, X₅, n have the same meaning as defined previously.

In another variant, when reacting the organic compound with structural element (I) as starting material 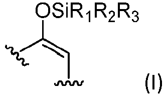 with Bis(trifluoromethyl)-peroxide (BTMP) in the presence of at least one HF scavenger and acetone or acetonitrile, preferably acetone, as the at least one organic solvent, an organic compound comprising the structural element (IV) with at least one trifluoromethoxy moiety 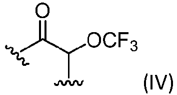 Is provided, i.e.α- trifluoromethoxylated ketones are obtained.

It is to be pointed out that instead of using acetone as organic solvent, it is also possible in certain cases to use dichloromethane (DCM), diethyl ether and/or nitromethane, preferably dichloromethane (DCM), as organic solvent in in combination with trifluoroacetic acid for hydrolysis of the silyl moiety to obtain *α*-Trifluoromethoxylated ketones.

When using acetone or acetonitrile, preferably acetone as organic solvent, in an embodiment the following conversions may result:

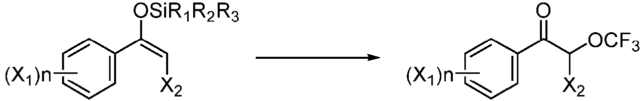

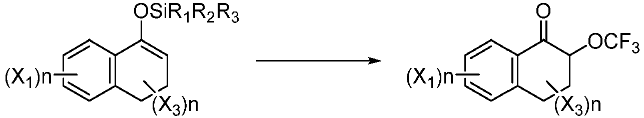

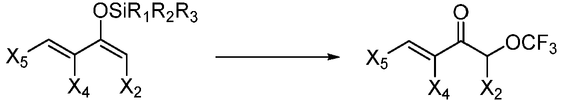

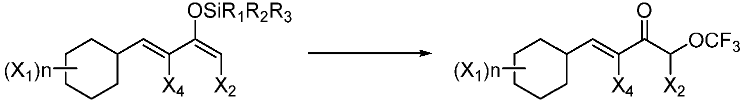

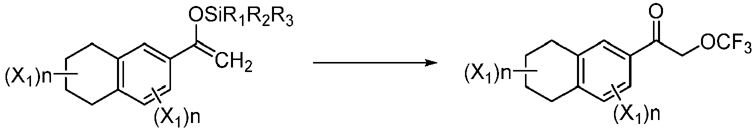

Wherein R₁, R₂, R₃, X₁, X₂, X₃, X₄, X₅, n have the same meaning as defined previously above.

In a further variant of the present method, when reacting the organic compound with structural element (II) as starting material 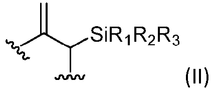 with Bis(trifluoromethyl)-peroxide (BTMP) in the presence of at least one HF scavenger and at least one organic solvent, preferably MeCN, an organic compound comprising the structural elements (V) with at least one trifluoromethoxy moiety 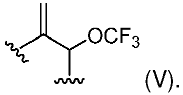 Is provided, i.e.trifluoromethoxylated allyl silanes.

In preferred embodiments the organic compound with structural element (II) used as starting material may be one of the following compounds: 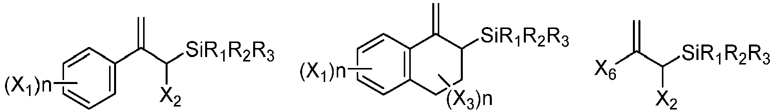 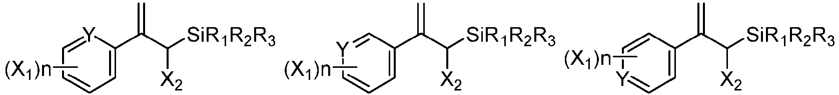 With
X₁ being H, alkyl, alkoxy, Halogen, -OCH₂C₆H₅ or -CₙF₂ₙ₊₁, -CN, an alkyl ring fused with C₆ aryl,
X₂ being H or alkyl,
X₃ being H or alkyl,
X₆ being H, or alkyl,
n being 0-5, and
Y being, N, S, or O.

In a preferred embodiment,
X₁ being H, C₁-C₆ alkyl, such as methyl, ethyl, propyl, butyl, C₁-C₆ alkoxy, F, Cl, Br, I, -OCH₂C₆H₅ or -CₙF₂ₙ₊₁, -CF₃, -C₂F₅, -CN, C5-C6 alkyl ring fused with the C6 aryl,
X₂ being H or C₁-C₆ alkyl,
X₃ being H or C₁-C₆ alkyl,
X₆ being H, or C₁-C₈ alkyl,
n being 0, 1, 2, or 3, and
Y being N, S, or O.

In preferred embodiments the organic compound with structural element (II) used as starting material may be one of the following compounds:

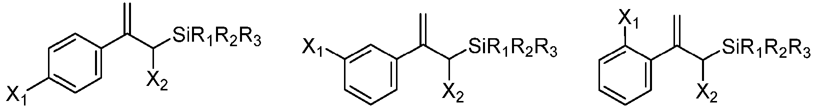

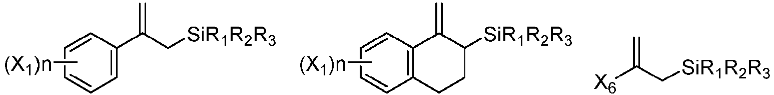

Wherein R₁, R₂, R₃, X₁, X₂, X₆, n have the same meaning as defined previously.

When using the organic compound with structural element (II) as starting material, the following conversions may result:

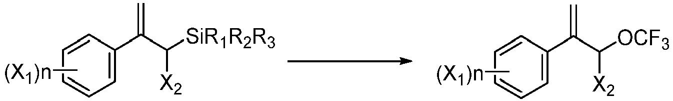

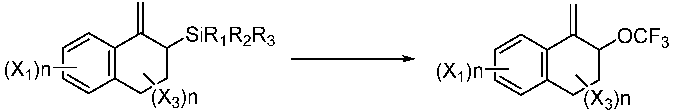

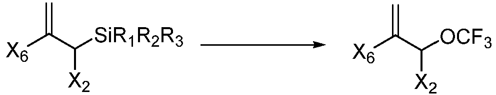

Wherein R₁, R₂, R₃, X₁, X₂, X₃, X₆, n have the same meaning as defined previously.

As shown previously, the present method allows the synthesis of organic compounds comprising one of the following structural elements (III), (IV) or (V) with at least one trifluoromethoxy moiety

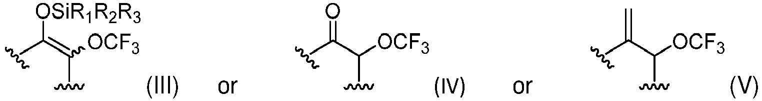

In one embodiment, at least one of the following compounds containing the structural element (III) with at least one trifluoromethoxy moiety (i.e. trifluoromethoxylated silyl enol ether) are provided:

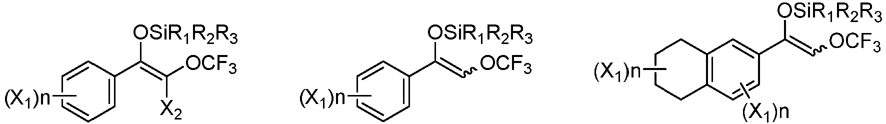

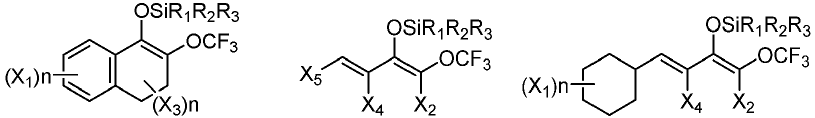

Wherein R₁, R₂, R₃, X₁, X₂, X₃, X₄, n have the same meaning as defined previously.

Specific embodiments of trifluoromethoxylated silyl enol ether are:

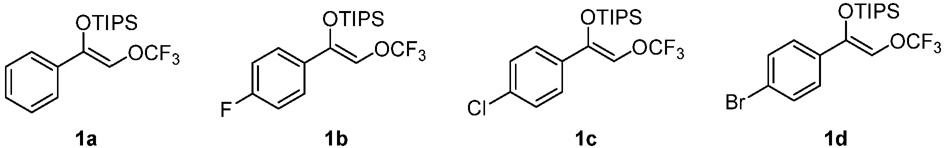

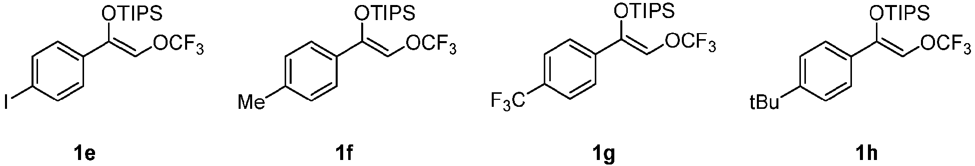

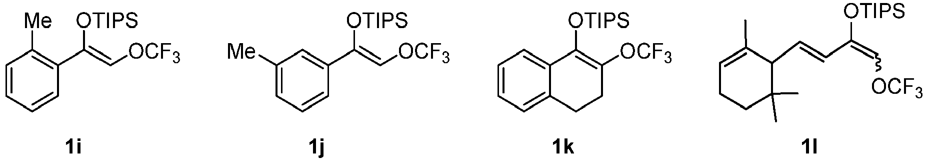

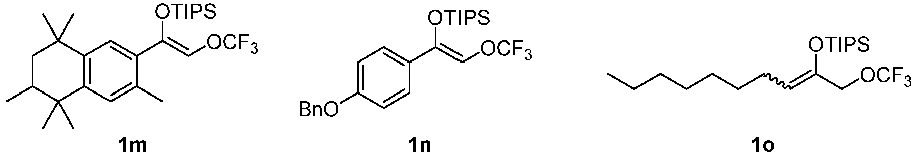

In another embodiment, at least one of the following compounds containing the structural element (IV) with at least one trifluoromethoxy moiety (i.e. *α*-Trifluoromethoxylated Ketones) are provided:

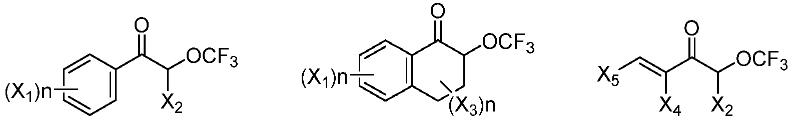

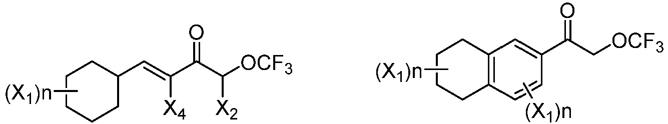

Wherein X₁, X₂, X₃, X₄, X₅, n have the same meaning as defined previously.

The following compounds and ketones are thereby exempted:

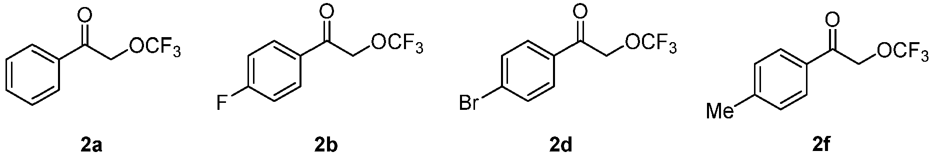

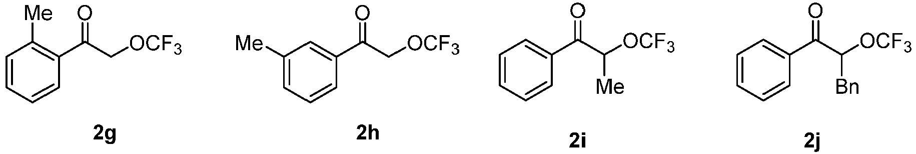

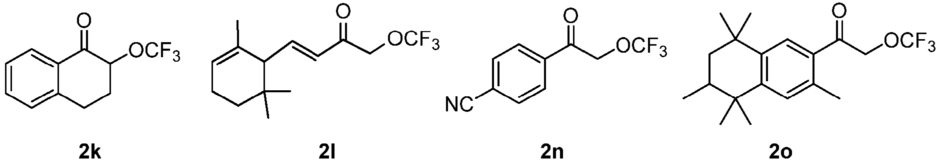

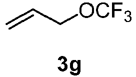

Specific embodiments of α-Trifluoromethoxylated Ketones are

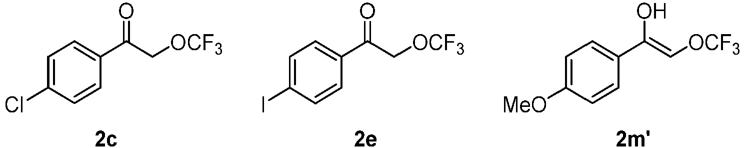

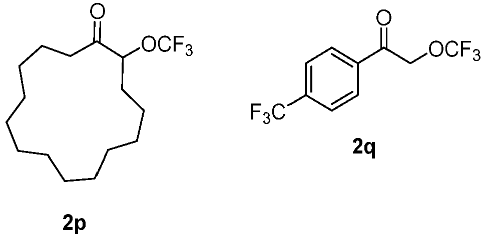

In yet a further embodiment, at least one of the following compounds containing the structural element (V) with at least one trifluoromethoxy moiety (i.e. trifluoromethoxylated allyl silanes) are provided:

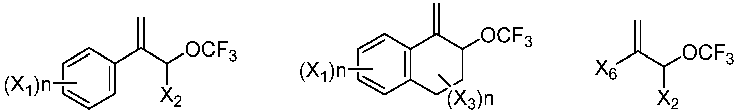

Wherein X₁, X₂, X₃, X₆, n have the same meaning as defined previously.

Specific embodiments of trifluoromethoxylated allyl silanes are:

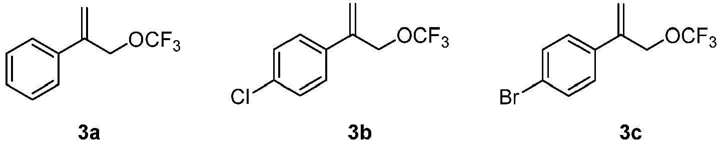

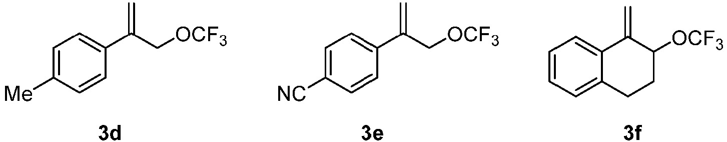

The invention is explained in more detail by means of the following figures and examples. It shows:
- Figure 1: a Reaction scheme for synthesizing α-Trifluoromethoxylated Ketones;
- Figure 2: a Reaction scheme for synthesizing trifluoromethoxylated silyl enol ether; and
- Figure 3: a Reaction scheme for synthesizing trifluoromethoxylated allyl silanes.

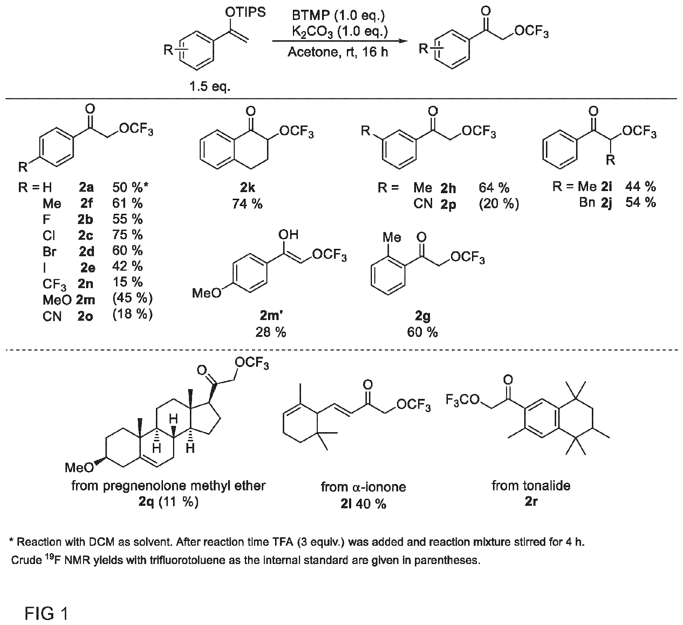

As illustrated in the reaction scheme of Figure 1, the present method allows the synthesis of different α-trifluoromethoxylated ketones, whereby common substituents on the aromatic ring such as alkyl moieties, ethers and halogens are well tolerated and lead to moderate to good isolated yields of up to 75%. Interestingly, the reaction of the methoxy-substituted TIPS enol ether gave not only the desired ketone 2m but also the stable enol 2m' in moderate yield. Products with substitutions in ortho or meta position, as well as with residues at the 2-position of the starting material could also be obtained in good yields. The reactions with strongly electron-withdrawing groups on the aromatic ring gave moderate yields. In order to evaluate a possible late-stage application of the method, complex and biologically active structures were investigated for their reactivity towards BTMP. The product **2l** starting from α-ionone could be synthesized in a moderate yield of 40 %.

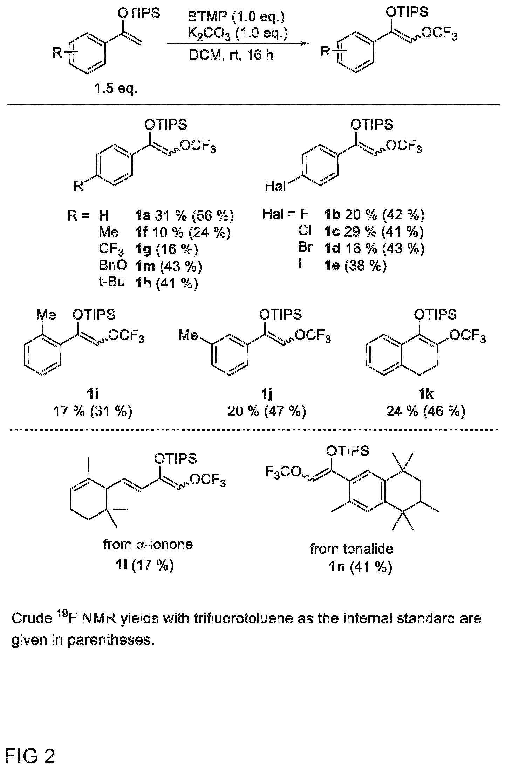

Similar to the method for the synthesis of OCF₃ ketones in Figure 1, the synthesis of trifluoromethoxylated silyl enol ether (DCM method, as illustrated in Figure 2) shows good tolerance to common substituents and substitution patterns. Halogens and alkyl groups give moderate yields and electron-withdrawing and -donating substituents are also tolerated. However, the success of the method seems to depend more on the stability of the enol ether. Substitution of the 2-position of the enol ether also seems to play a role in the formation of the product. The reaction of the enol ether starting from α-tetralone gave a yield of 24% for the product **1k**. Finally, OCF₃ enol ethers of the compounds, starting from the fragrance and natural products tonalide and α-ionone, could be obtained in moderate ¹⁹F NMR yields.

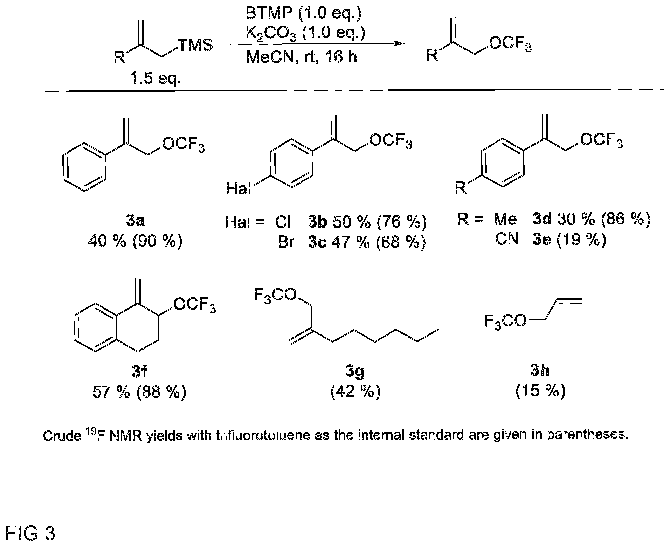

Figure 3 illustrates the general synthesis scheme for obtaining trifluoromethoxylated allyl silanes. Reaction conditions using trimethyl(2-phenylallyl) silane were tested. The conditions were investigated within a selected scope. Both halogens and alkyl substituents on the aromatic ring gave very good ¹⁹F NMR yields, with moderate to good isolated yields due to the volatility of some compounds. The allyl silane starting from *α*-tetralone also gave a very good ¹⁹F NMR yield of 88% and a good isolated yield of 57 % of product **3f.** Electron withdrawing substituents were tolerated and the aliphatic allyl OCF₃ products **3g** and **3h** could be obtained in moderate ¹⁹F NMR yields.

### 1 General Information

All purchased chemicals were used without further treatment.

Thin-layer chromatography was performed on silica gel coated aluminium plates ALUGRAM^{®} Xtra SIL G/UV254 (Macherey-Nagel). The product spots were detected by UV light (254 nm) or as permanganate stains. Flash column chromatography was performed with silica gel 60 M (0.040-0.063 mm, 230-400 mesh, Macherey-Nagel).

¹H, ¹⁹F and ¹³C NMR spectra were acquired on a JEOL ECS 400 (400 MHz), JEOL ECZ 400 (400 MHz), JEOL ECX 400 (400 MHz), JEOL Avance 500 (500 MHz), JEOL ECP 500 (500 MHz), Varian INOVA 600 (600 MHz) or a Bruker Avance 700 (700 MHz) and analysed on MestReNova 14.3.0. Chemical shifts (δ) are reported in parts per million (ppm) relative to tetramethyl silane (TMS) and coupling constants (J) are presented in hertz (Hz). CD₃CN or CDCl₃ are used as deuterated solvent and the residual solvent signals are used as reference in the ¹H and ¹³C NMR spectra. ¹⁹F NMR spectra are not calibrated by an internal reference. ¹⁹F NMR yields were measured using CF₃-toluene as an internal standard.

High-resolution mass spectra were measured with an Agilent (6210 ESI-TOF; 4 µL/min, 1.0 bar, 4 kV) instrument or a Varian MAT (MAT 711, electron energy 80 eV). Gas Chromatography mass spectra were measured with a Saturn 2100 GC-MS.

Infrared spectra were measured with a NICOLET spectrometer (iS10) equipped with an ATR unit (NICOLET SMART DuraSamplIR).

Analytical HPLC was performed in isocratic mode with a Smartline system from Knauer (Berlin, Germany), equipped with pump 1000, degasser, autosampler 3950 and variable wavelength UV detector 2500. The stationary phase usually was a pre-packed RP-18 column (RSC-Gel ec, 5 µm, 125 x 4.6 mm) from Richard Sauerbrey Chromatographie (Reinhardshagen, Germany). Additionally, for two compounds also a PGC column (Hypercarb, 5µm, 125 x 4.6 mm) from Thermo Scientific was employed. UV detection was performed at 210 nm. The flow was 1.5 mL·min-1. Eluents were degassed MeOH-water or MeCN-water mixtures, given as % (v/v) MeOH or MeCN, respectively.

Preparative HPLC was performed on an isocratic system equipped with a Shimadzu LC-8A pump, Shimadzu CBM-20A controller, variable wavelength UV detector from Knauer and a Rheodyne injector with 10 mL sample loop. Stationary phase usually was a RP-18 column (RSC-Gel ec, 5 µm, 250 x 32 mm) from Richard Sauerbrey Chromatographie (Reinhardshagen, Germany). Additionally, for two compounds also a preparative PGC column (Hypercarb, 5µm, 150 x 21 mm) from Thermo Scientific was employed. HPLC runs were performed with a total flow rate of eigther 40mL·min-1 (RP-18) or 20mL·min-1 (PGC) with UV detection at 210 nm. The purity of collected fractions was determined by analytical HPLC.

### 2 Trifluoromethoxylation of Silyl Enol Ethers

### 2.1 Method A: Synthesis of Trifluoromethoxylated Silyl Enol Ethers

The silyl enol ether (1.5 equiv., 0.75 mmol) and K₂CO₃ (1.0 equiv., 0.5 mmol, 69 mg) were added to a 15 mL Schlenk-pressure tube with DCM (2.5 mL). The mixture was frozen with liquid N₂ and degassed using freeze-pump-thaw technique. BTMP (1.0 equiv., 0.5 mmol, 85 mg) was condensed in, the reaction mixture was allowed to warm to rt and stirred vigorously at rt for 16 h. The pressure tube was opened carefully in the back of the fume hood to release possible excess gas.

**Crude NMR yields:** Trifluoro toluene (1.0 equiv, 0.5 mmol, 61 µL) and CDCl₃ (1 mL) was added to the reaction mixture. Up to 0.8 mL of the crude mixture was filtered through cotton into an NMR tube for ¹⁹F NMR analysis.

**Isolation:** The products were obtained after prep. HPLC (RP-18, MeOH/H₂O or MeCN/H₂O) as colourless oils.

### 2.2 Analytical Data for Trifluoromethoxylated Silyl Enol Ethers

Triisopropyl((1-phenyl-2-(trifluoromethoxy)vinyl)oxy)silane **1a**

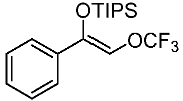

According to the general method A enol ether **1a** (270 mg, 0.75 mmol, 31 %) was purified by repeated preparative HPLC using two different solvent systems (first 80% MeCN, second 90% MeOH) and obtained from the corresponding silyl enol ether as a colourless oil on a 2.4 mmol (BTMP) scale.

**¹H NMR** (400 MHz, CDCl₃) δ = 7.56 - 7.47 (m, 2H), 7.44 - 7.35 (m, 3H), 6.46 (s, 1H), 1.24 (dq, *J=* 13.1, 7.1 Hz, 3H), 1.12 (d, *J =* 7.2 Hz, 18H).**¹⁹F NMR** (377 MHz, CDCl₃) δ = -60.4. ¹³C **NMR** (176 MHz, CDCl₃) δ = 142.7, 135.6, 128.8, 128.5, 125.7, 121.0 (q, *J =* 257 Hz), 118.8 (q, *J =* 4 Hz), 17.9, 13.4. **IR (ATR):** *ṽ* [cm⁻¹] = 3108 (w), 3065 (w), 2947 (m), 2869 (m), 1668 (m), 1465 (m), 1364 (m), 1259 (s), 1214 (s), 1160 (s), 1137 (s), 1081 (m), 1067 (m), 882 (m), 850 (m), 684 (s), 599 (s). **HRMS (EI):** m/z calculated for [C₁₈H₂₇F₃O₂Si]⁺ ([M]⁺): 360.1732, measured: 360.1705.

((1-(4-fluorophenyl)-2-(trifluoromethoxy)vinyl)oxy)triisopropylsilane **1b**

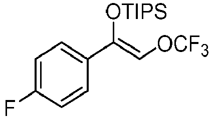

According to the general method A enol ether **1b** (38 mg, 0.1 mmol, 20 %) was purified by preparative HPLC (85% MeOH) and obtained from the corresponding silyl enol ether as a colourless oil.

**¹H NMR** (600 MHz, CDCl₃) δ = 7.49 - 7.40 (m, 2H), 7.09 - 6.98 (m, 2H), 6.34 (s, 1H), 1.25 - 1.11 (m, 3H), 1.06 (d, *J =* 7.0 Hz, 18H). ¹⁹F **NMR** (565 MHz, CDCl₃) δ = -60.4 (OCF₃), -112.7 (tt, *J =* 9, 5 Hz, F). ¹³C **NMR** (176 MHz, CDCl₃) δ = 163.1 (d, *J =* 249 Hz), 141.9, 131.8 (d, *J =* 3 Hz), 127.6 (d, *J* = 8. Hz), 121.0 (q, *J* = 257 Hz), 118.6 (q, *J* = 4 Hz), 115.5 (d, *J* = 22 Hz), 17.9, 13.4. **IR (ATR):** *ṽ* [cm⁻¹] = 3112 (w), 3054 (w), 2947 (m), 2869 (m), 1671 (m), 1605 (m), 1509 (m), 1365 (m), 1262 (s), 1213 (s), 1156 (s), 1139 (s), 1079 (m), 1067 (m), 882 (m), 839 (s), 684 (s), 567 (s). **HRMS (EI):** m/z calculated for [C₁₈H₂₆F₄O₂Si]⁺ ([M]⁺):378.1638, measured: 378.1668.

((1-(4-chlorophenyl)-2-(trifluoromethoxy)vinyl)oxy)triisopropylsilane **1c**

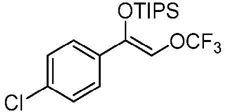

According to the general method A enol ether **1c** (57 mg, 0.15 mmol, 29 %) was purified by preparative HPLC (90% MeOH) and obtained from the corresponding silyl enol ether as a colourless oil.

**¹H NMR** (400 MHz, CDCl₃) δ = 7.44 - 7.36 (m, 2H), 7.36 - 7.28 (m, 2H), 6.39 (s, 1H), 1.25 - 1.12 (m, 3H), 1.06 (d, *J =* 7.1 Hz, 18H). **¹⁹F NMR** (377 MHz, CDCl₃) δ = -60.4. **¹³C NMR** (151 MHz, CDCl₃) δ = 141.7, 134.6, 134.2, 128.8, 126.9, 121.0 (q, *J =* 258 Hz), 119.0 (q, *J =* 4 Hz), 17.9, 13.4. **IR (ATR):** *ṽ* [cm⁻¹] = 3116 (w), 2947 (m), 2869 (m), 1669 (m), 1598 (w), 1367 (m), 1259 (s), 1212 (s), 1161 (s), 1140 (s), 1092 (m), 1079 (m), 1013 (m), 882 (m), 851 (m), 833 (s), 684 (m). **HRMS (EI):** m/z calculated for [C₁₈H₂₆ClF₃O₂Si]⁺ ([M]⁺): 394.1343, measured: 394.1315.

((1-(4-bromophenyl)-2-(trifluoromethoxy)vinyl)oxy)triisopropylsilane **1d**

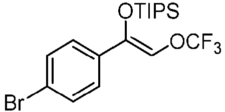

According to the general method A enol ether **1d** (36 mg, 0.08 mmol, 16 %) was purified by repeated preparative HPLC using two different solvent systems (first 92% MeOH, second 85% MeCN) and obtained from the corresponding silyl enol ether as a colourless oil.

**¹H NMR** (400 MHz, CD₃CN) δ = 7.58 - 7.50 (m, 2H), 7.48 - 7.39 (m, 2H), 6.68 (s, 1H), 1.24 - 1.13 (m, 3H), 1.06 (d, *J=* 7.1 Hz, 18H). **¹⁹F NMR** (377 MHz, CD₃CN) δ = -60.9. **¹³C NMR** (101 MHz, CD₃CN) δ = 142.6, 135.3, 132.5, 128.4, 123.2, 121.8 (q, *J =* 256 Hz), 120.5 (q, *J =* 3.5 Hz), 18.1, 14.0. IR **(ATR):** *ṽ* [cm⁻¹] = 3116 (w), 2946 (m), 2869 (m), 1668 (m), 1591 (w), 1365 (m), 1258 (s), 1212 (s), 1161 (s), 1140 (s), 1092 (m), 1080 (m), 1009 (m), 882 (m), 850 (m), 830 (s), 684 (m), 611 (s). **HRMS (EI):** m/z calculated for [C₁₈H₂₆BrF₃O₂Si]⁺ ([M]⁺): 438.0838, measured: 438.0817.

((1-(4-iodophenyl)-2-(trifluoromethoxy)vinyl)oxy)triisopropylsilane **1e**

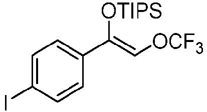

According to the general method enol ether **1e** (29 mg, 0.06 mmol, 12 %) was purified by repeated preparative HPLC using two different solvent systems (first 93% MeOH, second 85% MeCN) and obtained from the corresponding silyl enol ether as a colourless oil.

**¹H NMR** (400 MHz, CD₃CN) δ = 7.77 - 7.72 (m, 2H), 7.32 - 7.28 (m, 2H), 6.68 (s, 1H), 1.23 - 1.13 (m, 3H), 1.06 (d, *J =* 7.0 Hz, 18H). **¹⁹F NMR** (377 MHz, CD₃CN) δ = -60.9. **¹³C NMR** (101 MHz, CD₃CN) δ = 142.7, 138.5, 135.8, 128.4, 121.8 (d, *J* = 256 Hz), 120.5 (q, *J* = 3.5 Hz), 94.8, 18.1, 14.0. **IR (ATR):** *ṽ* [cm⁻¹] = 3114 (w), 2946 (m), 2868 (m), 1668 (m), 1464 (m), 1365 (m), 1258 (s), 1212 (s), 1161 (s), 1139 (s), 1080 (s), 1004 (s), 882 (m), 849 (m), 826 (s), 724 (m), 683 (s), 608 (m). **HRMS (EI):** m/z calculated for [C₁₈H₂₆IF₃O₂Si]⁺ ([M]⁺): 486.0699, measured: 486.0670.

Triisopropyl((1-(p-tolyl)-2-(trifluoromethoxy)vinyl)oxy)silane **1f**

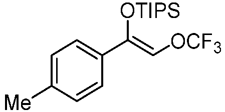

According to the general method A enol ether 1f (18 mg, 0.05 mmol, 10 %) was purified by repeated preparative HPLC using two different solvent systems (first 88% MeCN, second 90% MeOH) and obtained from the corresponding silyl enol ether as a colourless oil.

**¹H NMR** (400 MHz, CD₃CN) δ = 7.45 (dt, *J =* 8.2, 2.2 Hz, 2H), 7.28 - 7.20 (m, 2H), 6.62 (s, 1H), 2.38 (s, 3H), 1.30 - 1.15 (m, 3H), 1.11 (d, *J =* 7.0 Hz, 18H).**¹⁹F NMR** (377 MHz, CD₃CN) δ = -60.9. **¹³C NMR** (101 MHz, CD₃CN) δ = 143.0, 139.1, 132.2, 129.1, 125.6, 121.0 (q, *J =* 255 Hz), 118.7 (q, *J =* 3 Hz), 20.2, 17.2, 13.1. IR **(ATR):** *ṽ* [cm⁻¹] = 3118 (w), 3030 (w), 2946 (m), 2869 (m), 1670 (m), 1464 (m), 1363 (m), 1259 (s), 1214 (s), 1158 (s), 1136 (s), 1078 (s), 1067 (s), 1018 (m), 882 (m), 854 (m), 821 (s), 725 (m), 683 (s), 568 (m). **HRMS (EI):** m/z calculated for [C₁₉H₂₉F₃O₂Si]⁺ ([M]⁺): 374.1889, measured: 374.1898.

Triisopropyl((2-(trifluoromethoxy)-1-(4-(trifluoromethyl)phenyl)vinyl)oxy)silane **1g**

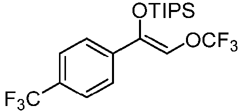

According to the general method enol ether **1g** (11 mg, 0.03 mmol, 5 %) was purified by repeated preparative HPLC using two different solvent systems (first 90% MeOH, second 85% MeCN) and obtained from the corresponding silyl enol ether as a colourless oil.

**¹H NMR** (400 MHz, CD₃CN) δ = 7.72 - 7.68 (m, 4H), 6.80 (s, 1H), 1.24 - 1.16 (m, 3H), 1.07 (d, *J* = 7.3 Hz, 18H). **¹⁹F NMR** (377 MHz, CD₃CN) δ = -60.9 (OCF₃), -63.1 (CF₃). **¹³C NMR** (151 MHz, CD₃CN) δ = 141.2, 139.2, 129.9 (q, *J* = 32 Hz), 126.1, 125.5 (q, *J* = 4 Hz), 124.3 (d, *J =* 272.0 Hz), 122.6 (q, *J =* 258 Hz), 120.7 (q, *J =* 3 Hz), 17.2, 13.1. **IR (ATR):** *ṽ* [cm⁻¹] = 3116 (w), 2948 (m), 2870 (m), 1669 (m), 1465 (w), 1324 (s), 1261 (s), 1214 (s), 1165 (s), 1128 (s), 1110 (s), 1082 (s), 1067 (s), 1015 (m), 882 (m), 846 (s), 721 (m), 685 (m), 613 (m). **HRMS (EI):** m/z calculated for [C₁₉H₂₆F₆O₂Si]⁺ ([M]⁺): 428.1606, measured: 428.1594.

((1-(4-(tert-butyl)phenyl)-2-(trifluoromethoxy)vinyl)oxy)triisopropylsilane **1h**

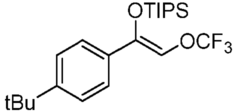

According to the general method A enol ether **1h** (83 mg, 0.2 mmol, 41 %) was purified by preparative HPLC (RP-18 with 81% MeOH) and obtained from the corresponding silyl enol ether as a colourless oil.

**¹H NMR** (400 MHz, CD₃CN) δ = 7.51 - 7.44 (m, 4H), 6.64 (s, 1H), 1.34 (s, 9H), 1.28 - 1.20 (m, 3H), 1.11 (d, *J* = 7.2 Hz, 18H). **¹⁹F NMR** (377 MHz, CD₃CN) δ = -60.9. **¹³C NMR** (101 MHz, CD₃CN) δ = 152.2, 142.8, 132.2, 125.4, 125.3, 121.0 (q, *J =* 255.5 Hz), 118.7 (q, *J =* 3.5 Hz), 34.3, 30.5, 17.2, 13.1.

Triisopropyl((1-(o-tolyl)-2-(trifluoromethoxy)vinyl)oxy)silane **1i**

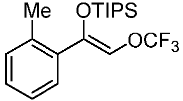

According to the general method A enol ether **1i** (33 mg, 0.09 mmol, 18 %) was purified by repeated preparative HPLC using two different solvent systems (first 88% MeCN, second 90% MeOH) and obtained from the corresponding silyl enol ether as a colourless oil.

**¹H NMR** (400 MHz, CD₃CN) δ = 7.32 - 7.26 (m, 2H), 7.25 - 7.22 (m, 1H), 7.18 (td, J= 7.5, 1.4 Hz, 1H), 6.18 (s, 1H), 2.38 (s, 3H), 1.17 - 1.04 (m, 3H), 0.99 (d, *J* = 6.6 Hz, 18H). **¹⁹F NMR** (377 MHz, CD₃CN) δ = -60.9. **¹³C NMR** (101 MHz, CD₃CN) δ = 143.5, 137.5, 134.6, 130.4, 129.4, 129.2, 125.6, 121.0 (q, *J* = 255 Hz), 120.2 (q, *J =* 3 Hz), 22.1, 17.0, 12.8. **IR (ATR):** *ṽ* [cm⁻¹] = 3106 (w), 3067 (w), 3026 (w), 2946 (m), 2869 (m), 1673 (m), 1464 (m), 1356 (s), 1257 (s), 1216 (s), 1158 (s), 1133 (s), 1064 (s), 1045 (m), 882 (s), 856 (s), 768 (s), 683 (s), 605 (s). **HRMS (EI):** m/z calculated for [C₁₉H₂₉F₃O₂Si]⁺ ([M]⁺): 374.1889, measured: 374.1870.

Triisopropyl((1-(m-tolyl)-2-(trifluoromethoxy)vinyl)oxy)silane **1j**

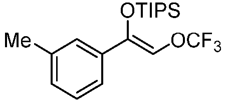

According to the general method A enol ether **1j** (38 mg, 0.1 mmol, 20 %) was purified by repeated preparative HPLC using two different solvent systems (first 88% MeCN, second 90% MeOH) and obtained from the corresponding silyl enol ether as a colourless oil.

**¹H NMR** (400 MHz, CD₃CN) δ = 7.38 - 7.29 (m, 2H), 7.26 (t, *J=* 7.5 Hz, 1H), 7.19 (d, *J=* 7.3 Hz, 1H), 6.61 (s, 1H), 2.33 (s, 3H), 1.26 - 1.11 (m, 3H), 1.07 (d, *J* = 6.9 Hz, 18H). **¹⁹F NMR** (377 MHz, CD₃CN) δ = -60.9. **¹³C NMR** (101 MHz, CD₃CN) δ = 143.0, 138.3, 135.0, 129.6, 128.4, 126.3, 122.8, 121.0 (q, *J =* 255.5 Hz), 119.1 (q, *J =* 3.5 Hz), 20.4, 17.2, 13.1. **IR (ATR):** *ṽ* [cm⁻¹] = 3106 (w), 3030 (w), 2946 (m), 2869 (m), 1669 (m), 1464 (w), 1360 (m), 1260 (s), 1219 (s), 1192 (s), 1158 (s), 1133 (s), 1080 (m), 881 (s), 851 (m), 788 (m), 721 (m), 684 (s), 603 (m). **HRMS (EI):** m/z calculated for [C₁₉H₂₉F₃O₂Si]⁺ ([M]⁺): 374.1889, measured: 374.1884.

Triisopropyl((2-(trifluoromethoxy)-3,4-dihydronaphthalen-1-yl)oxy)silane **1k**

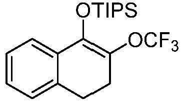

According to the general method A enol ether **1k** (46 mg, 0.12 mmol, 24 %) was purified by repeated preparative HPLC using two different solvent systems (first 91% MeOH, second 85% MeCN) and obtained from the corresponding silyl enol ether as a colourless oil.

**¹H NMR** (400 MHz, CD₃CN) δ = 7.48 (dd, *J= 7.1,* 1.8 Hz, 1H), 7.23 (dtd, J= 14.2, 7.3, 1.7 Hz, 2H), 7.19 - 7.15 (m, 1H), 2.90 (t, *J* = 7.1 Hz, 2H), 2.60 (dt, *J =* 7.2, 1.2 Hz, 2H), 1.32 - 1.21 (m, 3H), 1.10 (d, *J=* 7.4 Hz, 18H). **¹⁹F NMR** (377 MHz, CD₃CN) δ = -56.8. **¹³C NMR** (101 MHz, CD₃CN) δ = 140.2, 135.8, 133.8, 131.8, 128.9, 128.0, 127.4, 123.4, 122.0 (d, *J* = 256 Hz), 28.9, 26.0 (q, *J* = 1 Hz), 23.0, 18.2, 14.2. **IR (ATR):** *ṽ* [cm⁻¹] = 3071 (w), 3024 (w), 2946 (m), 2869 (m), 1670 (m), 1465 (m), 1327 (s), 1236 (s), 1200 (s), 1150 (s), 1093 (m), 1001 (m), 909 (m), 883 (m), 795 (m), 764 (s), 684 (s). **HRMS (EI):** m/z calculated for [C₂₀H₂₉F₃O₂Si]⁺ ([M]⁺): 386.1889, measured: 386.1859.

Triisopropyl(((1E,3E)-1-(trifluoromethoxy)-4-(2,6,6-trimethylcyclohex-2-en-1-yl)buta-1,3-dien-2-yl)oxy)silane **1l**

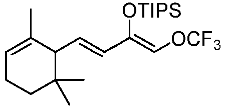

According to the general method enol ether **1l** (34 mg, 0.08 mmol, 16 %) was purified by preparative HPLC (95% MeOH) and obtained as a colourless oil.

**¹H NMR** (400 MHz, CD₃CN) δ = 6.27 (s, 1H), 5.91 (dd, J= 15.2, 9.4 Hz, 1H), 5.79 (d, *J=* 15.3 Hz, 1H), 5.45 - 5.40 (m, 1H), 2.27 - 2.20 (m, 1H), 2.05 - 1.96 (m, 2H), 1.55 (q, *J* = 1.9 Hz, 3H), 1.42 (dt, *J =* 13.2, 8.1 Hz, 1H), 1.29 - 1.16 (m, 4H), 1.10 (d, *J* = 7.2 Hz, 18H), 0.89 (s, 3H), 0.82 (s, 3H). **¹⁹F NMR** (377 MHz, CD₃CN) δ = -61.1. **¹³C NMR** (101 MHz, CD₃CN) δ = 142.2, 134.5, 133.7, 125.4, 122.1, 121.1 (q, *J* = 4 Hz), 121.7 (q, *J =* 255 Hz), 54.7, 33.0, 32.1, 27.9, 27.0, 23.7, 23.0, 18.2, 14.1. **IR (ATR):** *ṽ* [cm⁻¹] = 3106 (w), 3030 (w), 2946 (m), 2869 (m), 1669 (m), 1464 (w), 1360 (m), 1260 (s), 1219 (s), 1192 (s), 1158 (s), 1133 (s), 1080 (m), 881 (s), 851 (m), 788 (m), 721 (m), 684 (s), 603 (m). **HRMS (EI):** m/z calculated for [C₂₃H₃₉F₃O₂Si]⁺ ([M]⁺): 432.2671, measured: 432.2644.

(Z)-((1-(3,5,5,6,8,8-hexamethyl-5,6,7,8-tetrahydronaphthalen-2-yl)-2-(trifluoromethoxy)vinyl)oxy)triisopropylsilane **1m**

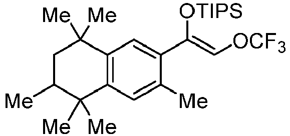

According to the general method enol ether 1m (66 mg, 0.13 mmol, 26 %) was purified by repeated preparative HPLC using two different solvent systems (first 95% MeOH, second 95% MeCN) and obtained as a colourless oil.

**¹H NMR** (400 MHz, CD₃CN) δ = 7.27 (s, 1H), 7.17 (s, 1H), 6.54 (q, *J =* 1.0 Hz, 1H), 2.27 (s, 3H), 1.87 (ddd, *J* = 13.0, 6.7, 2.6 Hz, 1H), 1.62 (t, *J =* 13.2 Hz, 1H), 1.39 (dd, *J =* 13.5, 2.6 Hz, 1H), 1.31 (s, 3H), 1.23 (s, 3H), 1.20 (s, 3H), 1.18 - 1.08 (m, 3H), 1.05 (s, 3H), 1.03 - 1.00 (m, 18H), 1.00 (s, 3H). **¹⁹F NMR** (377 MHz, CD₃CN) δ = -61.3. **¹³C NMR** (101 MHz, CD₃CN) δ = 149.7, 147.9, 142.6, 134.3, 132.1, 129.7, 128.3, 123.3 (q, *J =* 3 Hz), 121.8 (q, *J =* 254 Hz), 44.1, 38.3, 35.4, 34.6, 32.3, 32.0, 28.8, 25.0, 19.3, 18.0, 17.0, 13.3. **IR (ATR):** *ṽ* [cm⁻¹] = 3028 (w), 2963 (m), 2869 (m), 1463 (m), 1364 (w), 1271 (m), 1232 (s), 1214 (s), 1157 (s), 1140 (s), 997 (w), 909 (m), 882 (m), 823 (m), 686 (m), 660 (m). **HRMS (EI):** m/z calculated for [C₂₈H₄₅F₃O₂Si]⁺ ([M+]⁺): 498.3141, measured:498.3123.

(Z)-((1-(4-(benzyloxy)phenyl)-2-(trifluoromethoxy)vinyl)oxy)triisopropylsilane **1n**

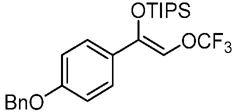

According to the general method enol ether **1n** (44 mg, 0.09 mmol, 19 %) was purified by repeated preparative HPLC using two different columns (first RP-18 with 91% MeOH, second PGC with 100% THF) and obtained as a colourless solid.

**¹H NMR** (400 MHz, CD₃CN) δ = 7.5 - 7.4 (m, 4H), 7.4 - 7.4 (m, 2H), 7.4 - 7.3 (m, 1H), 7.0 - 7.0 (m, 2H), 6.5 (s, 1H), 5.1 (s, 2H), 1.2 - 1.1 (m, 3H), 1.1 (d, *J =* 6.8 Hz, 18H). **¹⁹F NMR** (377 MHz, CD₃CN) δ = -60.9. **¹³C NMR** (101 MHz, CD₃CN) δ = 160.4, 143.8, 138.2, 129.5, 129.0, 128.7, 128.2, 121.9 (q, *J =* 255 Hz), 119.2 (q, *J* = 4 Hz), 115.7, 70.8, 18.2, 14.1. **IR (ATR):** *ṽ* [cm⁻¹] = 3112 (w), 2946 (m), 2868 (m), 1668 (w), 1607 (m), 1511 (m), 1363 (m), 1243 (s), 1215 (s), 1158 (s), 1136 (s), 1080 (s), 1012 (m), 882 (m), 833 (m), 733 (s), 684 (s), 609 (m). **HRMS (EI):** m/z calculated for [C₂₅H₃₃F₃O₃Si]⁺ ([M]⁺): 466.2151, measured: 466.2122.

triisopropyl((1-(trifluoromethoxy)dec-1-en-2-yl)oxy)silane **1o**

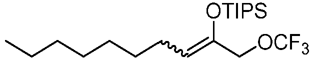

According to the general method A enol ether **1o** (22 mg, 0.06 mmol, 11 %) was purified by preparative HPLC (95% MeCN) and obtained as a mixture of two diastereomers (d.r. 1:1) as a colourless oil.

**¹H NMR** (600 MHz, CD₃CN) δ = 4.95 (t, *J =* 7.9 Hz, 1H, ), 4.87 (t, *J =* 7.2 Hz, 1H), 4.48 (s, 2H), 4.37 (s, 2H), 2.06 (q, *J =* 7.2 Hz, 2H), 2.03 - 1.96 (m, 2H), 1.34 - 1.21 (m, 20H), 1.21 - 1.12 (m, 6H), 1.07 (d, *J* = 7.2 Hz, 18H), 1.05 (d, *J =* 7.4 Hz, 18H), 0.85 (t, *J =* 7.1 Hz, 6H). **¹⁹F NMR** (377 MHz, CD₃CN) δ = -60.4, -60.6. **¹³C NMR** (151 MHz, CD₃CN) δ = 145.1, 144.5, 122.8 (d, *J* = 253 Hz), 122.6 (d, *J =* 253 Hz), 116.0, 114.4, 71.3 (q, *J =* 3.5 Hz), 66.2 (q, *J =* 3 Hz), 32.5, 32.5, 30.9, 30.0, 29.8, 29.7, 29.7, 29.5, 27.1, 26.0, 23.3, 23.2, 18.2, 18.2, 14.3, 13.9, 13.3. **IR (ATR):** *ṽ* [cm⁻¹] = 2927 (m), 2868 (m), 2262 (w), 1669 (w), 1464 (m), 1379 (w), 1256 (s), 1213 (s), 1197 (s), 1140 (s), 1015 (m), 1000 (m), 882 (m), 681 (s). **HRMS (EI):** m/z calculated for [C₂₀H₃₉F₃O₂Si]⁺ ([M]⁺): 396.2671, measured: 396.2679.

### 2.3 Synthesis of α-Trifluoromethoxylated Ketones

**Method B:** The silyl enol ether (1.5 equiv., 0.75 mmol) and K₂CO₃ (1.0 equiv., 0.5 mmol, 69 mg) were added to a 15 mL Schlenk-pressure tube with DCM (2.5 mL). The mixture was frozen with liquid N₂ and degassed using freeze-pump-thaw technique. BTMP (1.0 equiv., 0.5 mmol, 85 mg) was condensed in, the reaction mixture was allowed to warm to rt and stirred vigorously at rt for 16 h. The pressure tube was opened carefully in the back of the fume hood to release possible excess gas. Trifluoroacetic acid (3.0 equiv., 1.5 mmol, 244 µL) was added and the resulting mixture was stirred 4 h at rt.

**Crude NMR yields:** Trifluoro toluene (1.0 equiv, 0.5 mmol, 61 µL) and CDCl₃ (1 mL) was added to the reaction mixture. Up to 0.7 mL of the crude mixture was filtered through cotton into an NMR tube for ¹⁹F NMR analysis.

**Isolation:** The solvent was evaporated under reduced pressure and the products were obtained after column chromatography (SiO₂, pentane/DCM).

1-phenyl-2-(trifluoromethoxy)ethan-1-one **2a**

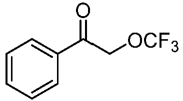

According to the general method B ketone **2a** (51 mg, 0.25 mmol, 50 %) was obtained from the corresponding silyl enol ether as a colourless oil.

**¹H NMR** (400 MHz, CDCl₃) δ = 7.93 - 7.87 (m, 2H), 7.67 - 7.61 (m, 1H), 7.55 - 7.47 (m, 2H), 5.18 (s, 2H). **¹⁹F NMR** (377 MHz, CDCl₃) δ = -61.0. **¹³C NMR** (101 MHz, CDCl₃) δ = 190.3, 134.5, 133.8, 129.2, 128.0, 121.8 (q, *J =* 257 Hz), 68.4 (q, *J =* 3 Hz).

The characterization data agree with literature values (O. Marrec, T. Billard, J.-P. Vors, S. Pazenok, B. R. Langlois, Adv Synth Catal 2010, 352, 2831-2837).

**Method C:** The silyl enol ether (1.5 equiv., 0.75 mmol) and K₂CO₃ (1.0 equiv., 0.5 mmol, 69 mg) were added to a 15 mL Schlenk-pressure tube with Acetone (2.5 mL). The mixture was frozen with liquid N₂ and degassed using freeze-pump-thaw technique. BTMP (1.0 equiv., 0.5 mmol, 85 mg) was condensed in, the reaction mixture was allowed to warm to rt and stirred vigorously at rt for 16 h. The pressure tube was opened carefully in the back of the fume hood to release possible excess gas.

**Crude NMR yields:** Trifluoro toluene (1.0 equiv, 0.5 mmol, 61 µL) and CDCl₃ (1 mL) was added to the reaction mixture. Up to 0.8 mL of the crude mixture was filtered through cotton into an NMR tube for ¹⁹F NMR analysis.

**Isolation:** The solids were filtered off and the solvent was evaporated under reduced pressure. The products were obtained after column chromatography (SiO₂, pentane/DCM).

### 2.4 Analytical Data for α-Trifluoromethoxylated Ketones

1-(4-fluorophenyl)-2-(trifluoromethoxy)ethan-1-one **2b**

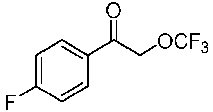

According to the general method C ketone **2b** (61 mg, 0.275 mmol, 55 %) was obtained from the corresponding silyl enol ether as a colourless oil.

**¹H NMR** (400 MHz, CDCl₃) δ = 7.99 - 7.92 (m, 2H), 7.24 - 7.15 (m, 2H), 5.13 (s, 2H). **¹⁹F NMR** (377 MHz, CDCl₃) δ = -61.0 (OCF₃), -102.3 (tt, *J =* 8, 5 Hz, 1F). **¹³C NMR** (101 MHz, CDCl₃) δ = 188.9, 166.5 (d, *J =* 257 Hz), 130.9 (d, *J =* 10 Hz), 130.4 (d, *J =* 257 Hz), 121.8 (q, *J =* 257 Hz), 116.5 (d, *J =* 22 Hz), 68.3 (q, *J =* 3 Hz).

The characterization data agree with literature values (T. Duhail, T. Bortolato, J. Mateos, E. Anselmi, B. Jelier, A. Togni, E. Magnier, G. Dagousset, L. Dell'Amico, Organic Letters 2021, 23, 7088-7093).

1-(4-chlorophenyl)-2-(trifluoromethoxy)ethan-1-one **2c**

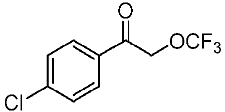

According to the general method C ketone **2c** (90 mg, 0.375 mmol, 75 %) was obtained from the corresponding silyl enol ether as a colourless solid.

**¹H NMR** (400 MHz, CDCl₃) δ = 7.84 (d, *J =* 8.7 Hz, 2H), 7.47 (d, *J =* 8.6 Hz, 2H), 5.14 (s, 2H). **¹⁹F NMR** (377 MHz, CDCl₃) δ = -61.0. **¹³C NMR** (151 MHz, CDCl₃) δ = 189.3, 141.0, 132.1, 129.5, 129.5, 121.7 (q, *J =* 257 Hz), 68.3 (q, *J =* 3 Hz). **IR (ATR):** *ṽ* [cm⁻¹] = 3097 (w), 2950 (w), 2870 (w), 1708 (m), 1592 (m), 1404 (m), 1264 (s), 1220 (s), 1142 (s), 1091 (s), 982 (s), 816 (s), 802 (s), 616 (m), 563 (s). **HRMS (EI):** m/z calculated for [C₉H₆ClF₃O₂]⁺ ([M]⁺): 238.0008, measured: 237.9977.

1-(4-bromophenyl)-2-(trifluoromethoxy)ethan-1-one **2d**

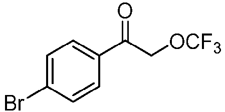

According to the general method C ketone **2d** (85 mg, 0.3 mmol, 60 %) was obtained from the corresponding silyl enol ether as a colourless solid.

**¹H NMR** (400 MHz, CDCl₃) δ = 7.82 - 7.74 (m, 2H), 7.69 - 7.64 (m, 2H), 5.13 (s, 2H). **¹⁹F NMR** (377 MHz, CDCl₃) δ = -61.0. **¹³C NMR** (176 MHz, CDCl₃) δ = 189.4, 132.4, 132.4, 129.7, 129.4, 121.6 (q, *J =* 257 Hz), 68.2 (q, *J =* 3 Hz).

The characterization data agree with literature values (T. Duhail, T. Bortolato, J. Mateos, E. Anselmi, B. Jelier, A. Togni, E. Magnier, G. Dagousset, L. Dell'Amico, Organic Letters 2021, 23, 7088-7093).

1-(4-iodophenyl)-2-(trifluoromethoxy)ethan-1-one **2e**

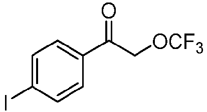

According to the general method C ketone **2e** (69 mg, 0.21 mmol, 42 %) was obtained from the corresponding silyl enol ether as a colourless solid.

**¹H NMR** (400 MHz, CDCl₃) δ = 7.92 - 7.84 (m, 2H), 7.66 - 7.56 (m, 2H), 5.11 (s, 2H). **¹⁹F NMR** (377 MHz, CDCl₃) δ = -61.0. **¹³C NMR** (101 MHz, CDCl₃) δ = 189.9, 138.5, 133.1, 129.3, 125.6, 121.7 (q, *J =* 257 Hz), 102.7, 68.2 (q, *J =* 3 Hz). **IR (ATR):** *ṽ* [cm⁻¹] = 3095 (w), 3057 (w), 2952 (w), 2879 (w), 1696 (s), 1580 (s), 1391 (m), 1374 (m), 1268 (s), 1197 (s), 1148 (s), 1096 (s), 1057 (s), 981 (s), 813 (s), 601 (m), 561 (s). **HRMS (EI):** m/z calculated for [C₉H₆F₃IO₂]⁺ ([M]⁺): 329.9365, measured: 329.9343.

1-(p-tolyl)-2-(trifluoromethoxy)ethan-1-one **2f**

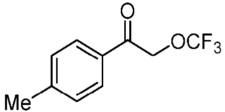

According to the general method C ketone **2f** (67 mg, 0.3 mmol, 61 %) was obtained from the corresponding silyl enol ether as a colourless oil.

**¹H NMR** (400 MHz, CDCl₃) δ = 7.80 (d, *J =* 8.3 Hz, 2H), 7.30 (d, *J =* 8.0 Hz, 2H), 5.15 (s, 2H), 2.43 (s, 3H). **¹⁹F NMR** (377 MHz, CDCl₃) δ = -60.9. **¹³C NMR** (101 MHz, CDCl₃) δ = 189.8, 145.5, 131.3, 129.8, 128.0, 121.7 (q, *J =* 256 Hz), 68.3 (q, *J =* 3 Hz), 21.8.

The characterization data agree with literature values (T. Duhail, T. Bortolato, J. Mateos, E. Anselmi, B. Jelier, A. Togni, E. Magnier, G. Dagousset, L. Dell'Amico, Organic Letters 2021, 23, 7088-7093).

1-(o-tolyl)-2-(trifluoromethoxy)ethan-1-one **2g**

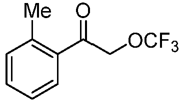

According to the general method C ketone **2g** (66 mg, 0.3 mmol, 60 %) was obtained from the corresponding silyl enol ether as a colourless solid.

**¹H NMR** (400 MHz, CDCl₃) δ = 7.54 (dd, *J =* 7.8, 1.4 Hz, 1H), 7.45 (td, *J =* 7.5, 1.4 Hz, 1H), 7.33 - 7.27 (m, 2H), 5.04 (s, 2H), 2.54 (s, 3H). **¹⁹F NMR** (377 MHz, CDCl₃) δ = -60.9. **¹³C NMR** (101 MHz, CDCl₃) δ = 193.5, 139.7, 133.7, 132.8, 132.6, 128.3, 126.0, 121.8 (q, *J =* 257 Hz), 69.3 (q, *J =* 3 Hz), 21.3.

The characterization data agree with literature values (T. Duhail, T. Bortolato, J. Mateos, E. Anselmi, B. Jelier, A. Togni, E. Magnier, G. Dagousset, L. Dell'Amico, Organic Letters 2021, 23, 7088-7093).

1-(m-tolyl)-2-(trifluoromethoxy)ethan-1-one **2h**

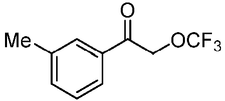

According to the general method C ketone **2h** (70 mg, 0.32 mmol, 64 %) was obtained from the corresponding silyl enol ether as a colourless oil.

**¹H NMR** (400 MHz, CDCl₃) δ = 7.75 - 7.65 (m, 2H), 7.49 - 7.42 (m, 1H), 7.39 (t, *J =* 7.6 Hz, 1H), 5.17 (s, 2H), 2.43 (s, 3H). **¹⁹F NMR** (377 MHz, CDCl₃) δ = -60.9. **¹³C NMR** (101 MHz, CDCl₃) δ = 190.4, 139.2, 135.3, 133.9, 129.0, 128.5, 125.2, 121.8 (q, *J=* 257 Hz), 68.4 (q, *J =* 3 Hz), 21.5.

The characterization data agree with literature values (T. Duhail, T. Bortolato, J. Mateos, E. Anselmi, B. Jelier, A. Togni, E. Magnier, G. Dagousset, L. Dell'Amico, Organic Letters 2021, 23, 7088-7093).

1-phenyl-2-(trifluoromethoxy)propan-1-one **2i**

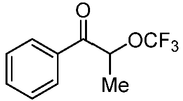

According to the general method C ketone **2i** (48 mg, 0.22 mmol, 44 %) was obtained from the corresponding silyl enol ether as a colourless oil.

**¹H NMR** (400 MHz, CDCl₃) δ = 8.00 - 7.93 (m, 2H), 7.68 - 7.58 (m, 1H), 7.56 - 7.46 (m, 2H), 5.49 (q, *J =* 7.0 Hz, 1H), 1.65 (d, *J =* 6.9 Hz, 3H). **¹⁹F NMR** (377 MHz, CDCl₃) δ = -58.8. **¹³C NMR** (151 MHz, CDCl₃) δ = 195.1, 134.2, 133.7, 129.1, 128.9, 121.6 (d, *J =* 257 Hz), 75.4 (q, *J* = 3 Hz), 18.8.

The characterization data agree with literature values (T. Duhail, T. Bortolato, J. Mateos, E. Anselmi, B. Jelier, A. Togni, E. Magnier, G. Dagousset, L. Dell'Amico, Organic Letters 2021, 23, 7088-7093).

1,3-diphenyl-2-(trifluoromethoxy)propan-1-one **2j**

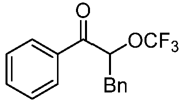

According to the general method C ketone **2j** (79 mg, 0.27 mmol, 54 %) was obtained from the corresponding silyl enol ether as a colourless oil.

**¹H NMR** (400 MHz, CDCl₃) δ = 7.94 - 7.89 (m, 2H), 7.66 - 7.58 (m, 1H), 7.52 - 7.46 (m, 2H), 7.33 - 7.25 (m, 3H), 7.25 - 7.20 (m, 2H), 5.51 (dd, *J =* 7.8, 5.3 Hz, 1H), 3.25 - 3.20 (m, 2H). **¹⁹F NMR** (377 MHz, CDCl₃) δ = -58.9. **¹³C NMR** (101 MHz, CDCl₃) δ = 194.7, 135.0, 134.2, 129.5, 129.1, 128.9, 128.8, 127.6, 121.4 (q, *J =* 257 Hz), 79.5 (q, *J =* 2 Hz), 53.6, 39.0.

The characterization data agree with literature values (T. Duhail, T. Bortolato, J. Mateos, E. Anselmi, B. Jelier, A. Togni, E. Magnier, G. Dagousset, L. Dell'Amico, Organic Letters 2021, 23, 7088-7093).

2-(trifluoromethoxy)-3,4-dihydronaphthalen-1 (2H)-one **2k**

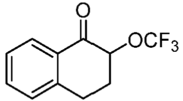

According to the general method C ketone **2k** (85 mg, 0.37 mmol, 74 %) was obtained from the corresponding silyl enol ether as a colourless oil.

**¹H NMR** (400 MHz, CDCl₃) δ = 8.06 (dd, *J =* 7.9, 1.5 Hz, 1H), 7.54 (td, *J =* 7.5, 1.5 Hz, 1H), 7.36 (ddt, *J =* 8.0, 7.4, 1.4 Hz, 1H), 7.32 - 7.23 (m, 1H), 4.84 (dd, *J =* 12.1, 4.8 Hz, 1H), 3.16 (dd, *J =* 8.1, 4.4 Hz, 2H), 2.54 (dq, *J =* 13.3, 4.5 Hz, 1H), 2.39 (tt, *J =* 12.6, 8.1 Hz, 1H). **¹⁹F NMR** (377 MHz, CDCl₃) δ = -58.5. **¹³C NMR** (151 MHz, CDCl₃) δ = 191.0, 142.8, 134.4, 131.3, 128.8, 128.3, 127.4, 121.9 (q, *J =* 256 Hz), 78.0 (q, *J =* 2 Hz), 30.2, 27.3.

The characterization data agree with literature values (H. Kondo, M. Maeno, K. Hirano, N. Shibata, Chem Commun 2018, 54, 5522-5525).

(E)-1-(trifluoromethoxy)-4-(2,6,6-trimethylcyclohex-2-en-1-yl)but-3-en-2-one **2l**

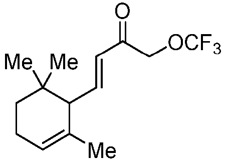

According to the general method C ketone **2l** (55 mg, 0.2 mmol, 40 %) was obtained from the corresponding silyl enol ether as a colourless oil.

**¹H NMR** (400 MHz, CDCl₃) δ = 6.86 (dd, *J =* 15.7, 9.8 Hz, 1H), 6.27 (d, *J =* 15.7 Hz, 1H), 5.52 (pd, *J =* 2.4, 1.4 Hz, 1H), 4.61 (s, 2H), 2.31 (d, *J =* 9.8 Hz, 1H), 2.09 - 1.98 (m, 2H), 1.55 (q, *J* = 1.9 Hz, 3H), 1.51 - 1.39 (m, 1H), 1.26 - 1.18 (m, 1H), 0.92 (s, 3H), 0.84 (s, 3H).¹⁹F **NMR** (377 MHz, CDCl₃) δ = -61.1. **¹³C NMR** (101 MHz, CDCl₃) δ = 191.0, 151.8, 131.3, 125.8, 123.4, 121.7 (q, *J =* 256 Hz), 69.5 (q, *J =* 3 Hz), 54.7, 32.8, 31.1, 28.0, 26.8, 23.1, 22.8.

The characterization data agree with literature values (T. Duhail, T. Bortolato, J. Mateos, E. Anselmi, B. Jelier, A. Togni, E. Magnier, G. Dagousset, L. Dell'Amico, Organic Letters 2021, 23, 7088-7093).

1-(4-methoxyphenyl)-2-(trifluoromethoxy)ethen-1-ol **2m'**

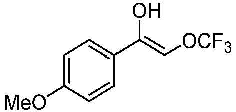

According to the general method B enol **2m'** (33 mg, 0.14 mmol, 28 %) was obtained from the corresponding silyl enol ether as a colourless oil.

**¹H NMR** (400 MHz, CDCl₃) δ = 8.10 - 7.99 (m, 2H), 7.19 (bs, 1H), 7.05 - 6.96 (m, 2H), 6.25 (s, 1H), 3.91 (s, 3H). **¹⁹F NMR** (377 MHz, CDCl₃) δ = -58.7. **¹³C NMR** (101 MHz, CDCl₃) δ = 184.1, 165.2, 132.6, 123.9, 120.7 (q, *J =* 261 Hz), 114.5, 93.8 (p, *J =* 3 Hz), 55.8. **IR (ATR):** *ṽ* [cm⁻¹] = 3085 (w), 3026 (w), 2929 (w), 2850 (w), 1688 (m), 1600 (s), 1515 (m), 1235 (s), 1166 (s), 1063 (m), 1027 (s), 983 (w), 875 (s), 839 (m), 700 (m), 588 (s). **HRMS (EI):** m/z calculated for [C₁₀H₉F₃O₃]⁺ ([M]⁺): 234.0504, measured: 234.0483.

4-(2-(trifluoromethoxy)acetyl)benzonitrile **2n**

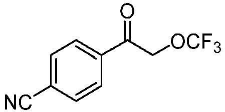

According to the general method B ketone 2n (20 mg, 0.09 mmol, 17 %) was obtained from the corresponding silyl enol ether as a white solid.

**¹H NMR** (400 MHz, CDCl₃) δ = 8.01 (d, *J =* 8.6 Hz, 2H), 7.83 (d, *J =* 8.5 Hz, 2H), 5.15 (s, 2H). **¹⁹F NMR** (377 MHz, CDCl₃) δ = -61.1. **¹³C NMR** (101 MHz, CDCl₃) δ = 189.5, 136.7, 132.9, 128.6, 121.6 (d, *J=* 257 Hz), 117.7, 117.5, 68.4 (q, *J =* 3 Hz).

The characterization data agree with literature values (T. Duhail, T. Bortolato, J. Mateos, E. Anselmi, B. Jelier, A. Togni, E. Magnier, G. Dagousset, L. Dell'Amico, Organic Letters 2021, 23, 7088-7093).

1-(3,5,5,6,8,8-hexamethyl-5,6,7,8-tetrahydronaphthalen-2-yl)-2-(trifluoromethoxy)ethan-1-one **2o**

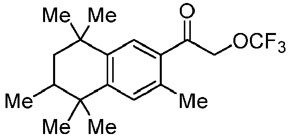

According to the general method B ketone **2o** (52 mg, 0.15 mmol, 43 %) was obtained from the corresponding silyl enol ether as a colourless oil.

**¹H NMR** (400 MHz, CDCl₃) δ = 7.54 (s, 1H), 7.32 (s, 1H), 5.09 (s, 2H), 2.56 (s, 3H), 1.94 (dqd, *J =* 13.4, 6.8, 2.6 Hz, 1H), 1.70 (t, *J =* 13.2 Hz, 1H), 1.47 (dd, *J =* 13.6, 2.7 Hz, 1H), 1.39 (s, 3H), 1.38 (s, 3H), 1.33 (s, 3H), 1.13 (s, 3H), 1.06 (d, *J* = 6.8 Hz, 3H). **¹⁹F NMR** (377 MHz, CDCl₃) δ = -60.8. **¹³C NMR** (101 MHz, CDCl₃) δ = 192.9, 151.6, 142.6, 136.4, 131.2, 127.1, 121.7 (d, *J =* 256 Hz), 69.2 (q, *J =* 3 Hz), 43.3, 38.1, 34.4, 34.1, 32.5, 31.9, 28.3, 24.7, 21.2, 16.8.

The characterization data agree with literature values (T. Duhail, T. Bortolato, J. Mateos, E. Anselmi, B. Jelier, A. Togni, E. Magnier, G. Dagousset, L. Dell'Amico, Organic Letters 2021, 23, 7088-7093).

2-(trifluoromethoxy)cyclopentadecan-1-one **2p**

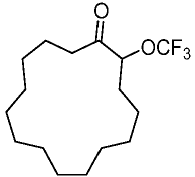

According to the general method B ketone **2p** (23 mg, 0.07 mmol, 24 %) was obtained from the corresponding silyl enol ether as a colourless oil.

**¹H NMR** (600 MHz, CDCl₃) δ = 4.50 (dd, *J* = 6.5, 5.2 Hz, 1H), 2.74 (ddd, *J* = 17.9, 8.1, 6.0 Hz, 1H), 2.43 (dt, *J* = 17.8, 6.3 Hz, 1H), 1.93 - 1.70 (m, 3H), 1.69 - 1.50 (m, 1H), 1.42 - 1.27 (m, 18H), 1.14 - 1.00 (m, 2H). **¹⁹F NMR** (377 MHz, CDCl₃) δ = -58.9. **¹³C NMR** (176 MHz, CDCl₃) δ = 208.0, 121.6 (q, *J* = 256 Hz), 82.2 (q, *J* = 2.5 Hz), 37.4, 31.7, 27.5, 27.2, 26.8, 26.8, 26.7, 26.6, 26.3, 26.3, 26.1, 22.6, 21.9. **IR (ATR):** *ṽ* [cm⁻¹] = 2927 (m), 2858 (m), 1724 (m), 1460 (m), 1362 (w), 1271 (s), 1222 (s), 1141 (s), 1061 (w), 881 (w), 626 (w). **HRMS (EI):** m/z calculated for [C₁₆H₂₇F₃O₂]⁺ ([M]⁺): 308.1963, measured: 308.1947.

2-(trifluoromethoxy)-1-(4-(trifluoromethyl)phenyl)ethan-1-one **2q**

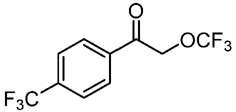

According to the general method B ketone **2q** (23 mg, 0.09 mmol, 17 %) was obtained from the corresponding silyl enol ether as a white solid.

**¹H NMR** (400 MHz, CDCl₃) δ = 8.1 - 8.0 (m, 2H), 7.8 - 7.8 (m, 2H), 5.2 (s, 2H). **¹⁹F NMR** (377 MHz, CDCl₃) δ = -61.1 (OCF₃), -63.3 (CF₃). **¹³C NMR** (151 MHz, CDCl₃) δ = 189.8, 136.5, 135.7 (q, *J* = 33 Hz), 128.6, 126.3 (q, *J* = 4 Hz), 123.4 (q, *J* = 273 Hz), 121.7 (q, *J* = 257 Hz), 68.5 (q, *J=* 3 Hz). **IR (ATR):** *ṽ* [cm⁻¹] = 3120 (w), 3075 (w), 2948 (w), 2872 (w), 1709 (m), 1516 (w), 1415 (m), 1326 (m), 1280 (m), 1218 (s), 1133 (s), 1111 (s), 1065 (s), 1016 (m), 993 (m), 846 (m), 831 (m), 614 (m), 601 (m). **HRMS (EI):** m/z calculated for [C₁₀H₆F₆O₂]⁺ ([M]⁺): 272.0272, measured: 272.0255.

### 3 Procedure and Analytical Data for the Trifluoromethoxylation of Allyl Silanes

The allyl silane (1.5 equiv., 0.75 mmol) and K₂CO₃ (1.0 equiv., 0.5 mmol, 69 mg) were added to a 15 mL Schlenk-pressure tube with MeCN (2.5 mL). The mixture was frozen with liquid N₂ and degassed using freeze-pump-thaw technique. BTMP (1.0 equiv., 0.5 mmol, 85 mg) was condensed in, the reaction mixture was allowed to warm to rt and stirred vigorously at rt for 16 h. The pressure tube was opened carefully in the back of the fume hood to release possible excess gas.

**Crude NMR yields:** Trifluoro toluene (1.0 equiv, 0.5 mmol, 61 µL) and CDCl₃ (1 mL) was added to the reaction mixture. Up to 0.8 mL of the crude mixture was filtered through cotton into an NMR tube for ¹⁹F NMR analysis.

**Isolation:** The solids were filtered off and the solvent was evaporated under reduced pressure. The products were obtained after column chromatography (SiO₂, pentane/DCM).

(3-(trifluoromethoxy)prop-1-en-2-yl)benzene **3a**

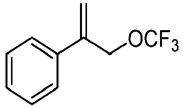

According to the general method allyl product 3a (40 mg, 0.2 mmol, 40 %) was obtained from the corresponding allyl silane as a colourless liquid.

**¹H NMR** (400 MHz, CDCl₃) δ = 7.46 - 7.42 (m, 2H), 7.42 - 7.32 (m, 3H), 5.64 (s, 1H), 5.47 (q, *J =* 1.1 Hz, 1H), 4.86 (d, *J =* 1.2 Hz, 2H). **¹⁹F NMR** (377 MHz, CDCl₃) δ = -60.4. **¹³C NMR** (101 MHz, CDCl₃) δ = 141.1, 137.4, 128.7, 128.5, 126.1, 121.8 (q, *J* = 255 Hz), 116.7, 68.8 (q, *J =* 3.5 Hz). **IR** (ATR): *ṽ* [cm⁻¹] = 3089 (w), 3061 (w), 3030 (w), 2962 (w), 2915 (w), 1254 (s), 1212 (s), 1134 (s), 1027 (m), 912 (m), 873 (m), 776 (s), 707 (s), 693 (s), 568 (m). **HRMS (EI):** m/z calculated for [C₁₀H₉F₃O]⁺ ([M]⁺): 202.0605, measured: 202.0619.

1-chloro-4-(3-(trifluoromethoxy)prop-1-en-2-yl)benzene **3b**

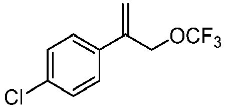

According to the general method allyl product **3b** (59 mg, 0.25 mmol, 50 %) was obtained from the corresponding allyl silane as a colourless liquid.

**¹H NMR** (400 MHz, CDCl₃) δ = 7.40 - 7.30 (m, 4H), 5.61 (s, 1H), 5.50 - 5.44 (m, 1H), 4.81 (d, *J* = 1.1 Hz, 2H). **¹⁹F NMR** (377 MHz, CDCl₃) δ = -60.5. **¹³C NMR** (101 MHz, CDCl₃) δ = 140.0, 135.7, 134.4, 128.9, 127.3, 121.7 (d, *J =* 256 Hz), 117.4, 68.6 (q, *J =* 3.5 Hz). **IR (ATR):** *ṽ* [cm⁻¹] = 3100 (w), 3048 (w), 2964 (w), 2907 (w), 1495 (m), 1252 (s), 1212 (s), 1137 (s), 1093 (m), 1013 (m), 921 (m), 875 (m), 832 (s), 770 (m), 737 (m), 558 (m). **HRMS (EI):** m/z calculated for [C₁₀H₈ClF₃O]⁺ ([M]⁺): 236.0191, measured: 236.0216.

1-bromo-4-(3-(trifluoromethoxy)prop-1-en-2-yl)benzene **3c**

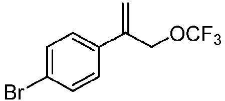

According to the general method allyl product **3c** (66 mg, 0.235 mmol, 47 %) was obtained from the corresponding allyl silane as a colourless liquid.

**¹H NMR** (400 MHz, CDCl₃) δ =7.58 - 7.53 (m, 2H), 7.37 - 7.33 (m, 2H), 5.68 (s, 1H), 5.54 - 5.52 (m, 1H), 4.86 (d, *J* = 1.2 Hz, 2H). **¹⁹F NMR** (377 MHz, CDCl₃) δ = -60.5. **¹³C NMR** (101 MHz, CDCl₃) δ = 140.2, 136.3, 131.9, 127.7, 122.6, 121.8 (q, *J=* 256 Hz), 117.6, 68.6 (q, *J =* 3 Hz). **IR (ATR):** *ṽ* [cm⁻¹] = 3098 (w), 3038 (w), 2966 (w), 2911 (w), 2858 (w), 1491 (m), 1254 (s), 1212 (s), 1136 (s), 1073 (m), 1008 (s), 919 (m), 875 (m), 828 (s), 760 (m), 734 (m), 581 (m). **HRMS (EI):** m/z calculated for [C₁₀H₈BrF₃O]⁺ ([M]⁺): 279.9718, measured: 279.9711.

1-methyl-4-(3-(trifluoromethoxy)prop-1-en-2-yl)benzene **3d**

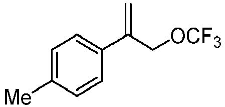

According to the general method allyl product **3d** (32 mg, 0.15 mmol, 30 %) was obtained from the corresponding allyl silane as a colourless liquid.

**¹H NMR** (400 MHz, CDCl₃) δ = 7.37 - 7.28 (m, 2H), 7.18 (d, *J* = 8.1 Hz, 2H), 5.59 (s, 1H), 5.40 (q, *J* = 1.1 Hz, 1H), 4.82 (d, *J* = 1.2 Hz, 2H), 2.36 (s, 3H). **¹⁹F NMR** (377 MHz, CDCl₃) δ = - 60.4. **¹³C NMR** (151 MHz, CDCl₃) δ = 140.7, 138.3, 134.4, 129.3, 125.8, 121.7 (q, *J=* 255 Hz), 115.7, 68.7 (q, *J* = 3 Hz), 21.1. **IR (ATR):** *ṽ* [cm⁻¹] = 3093 (w), 3059 (w), 3030 (w), 2956 (w), 2926 (w), 2895 (w), 1515 (m), 1251 (s), 1213 (s), 1138 (s), 1019 (m), 912 (m), 854 (s), 839 (s), 821 (s), 734 (m), 563 (m). **HRMS (EI):** m/z calculated for [C₁₁H₁₁F₃O]⁺ ([M]⁺): 216.0760, measured: 216.0762.

1-methylene-2-(trifluoromethoxy)-1,2,3,4-tetrahydronaphthalene **3f**

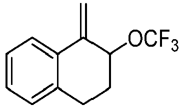

According to the general method allyl product **3f** (65 mg, 0.285 mmol, 57 %) was obtained from the corresponding allyl silane as a colourless liquid.

**¹H NMR** (400 MHz, CDCl₃) δ = 7.62 (dd, *J* = 7.3, 1.9 Hz, 1H), 7.22 (td, *J* = 6.7, 1.9 Hz, 2H), 7.16 - 7.11 (m, 1H), 5.70 (s, 1H), 5.33 (s, 1H), 5.04 (dd, *J* = 6.6, 2.7 Hz, 1H), 3.12 (ddd, J *=* 15.9, 9.6, 5.5 Hz, 1H), 2.87 (dt, *J* = 17.1, 5.5 Hz, 1H), 2.32 - 2.22 (m, 1H), 2.17 - 2.07 (m, 1H). **¹⁹F NMR** (377 MHz, CDCl₃) δ = -57.3. **¹³C NMR** (101 MHz, CDCl₃) δ = 140.7, 135.5, 132.1, 129.0, 128.5, 126.6, 125.1, 122.0 (d, *J* = 255 Hz), 112.4, 77.8 (q, *J* = 2 Hz), 29.3, 25.4. **IR (ATR):** *ṽ* [cm⁻¹] = 3098 (w), 3071 (w), 3020 (w), 2962 (w), 2935 (w), 2852 (w), 1487 (m), 1272 (s), 1219 (s), 1204 (s), 1183 (m), 1127 (s), 1028 (m), 908 (m), 846 (m), 773 (s), 743 (m), 735 (m), 576 (m). **HRMS (EI):** m/z calculated for [C₁₂H₁₁F₃O]⁺ ([M]⁺): 228.0762, measured: 228.0745.

3-(trifluoromethoxy)prop-1-ene **3g**

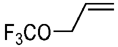

According to the general method allyl product **3g** (1.2 g, 9.5 mmol, 48 %) was obtained from allyltrimethylsilane on a 20 mmol scale as a liquid and colourless 1:1 mixture of TMSF and **3g.** The product was directly distilled out of the crude mixture via trap-to-trap and successive common distillation (bp = 26-32 °C).

**¹H NMR** (400 MHz, CDCl₃) δ = 6.0 - 5.8 (m, 1H), 5.4 (dq, *J* = 17.2, 1.4 Hz, 1H), 5.3 (dq, *J*= 10.4, 1.2 Hz, 1H), 4.5 (dt, *J* = 5.7, 1.4 Hz, 2H). **¹⁹F NMR** (377 MHz, CDCl₃) δ = -60.4. **¹³C NMR** (151 MHz, CDCl₃) δ = 130.8, 121.7 (d, *J* = 255 Hz), 119.6, 68.1 (q, *J* = 3 Hz). **GC-MS:** m/z calculated for [C₄H₅F₃O]⁺ ([M]⁺): 126.09, measured: 126.1.

The NMR data agree with the literature values (Patent: W. H. N. Ignatyev, M. Seidel,M. Bathe, J. Schroeter, K. Koppe, T. Meier, P. Barthen, W. Frank, (DE), WO2009141053, **2009**.)

## Claims

1. A synthesis method, preferably a catalyst free synthesis method for introducing a trifluoromethoxy moiety into at least one organic compound comprising the following steps:
- Providing at least one organic compound comprising at least one silyl moiety as part of the following structural elements (I) or (II) 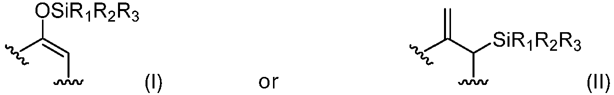 With R₁, R₂, R₃ being selected from C₁-C₄ Alkyl or C₆ Aryl, in particular -CH₃,-C₂H₅, -C₃H₇, -C₄H₉, - C₆H₅,
Wherein R₁, R₂, R₃ can be the same or different,
- Reacting the at least one organic compound with the structural elements (I) or (II) with Bis(trifluoromethyl)-peroxide (BTMP) in the presence of at least one HF scavenger and at least one organic solvent, and
- Obtaining at least one organic compound comprising at least one of the following structural elements (III), (IV) or (IV) with at least one trifluoromethoxy moiety

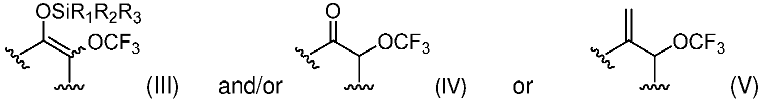

2. Method according to claim 1, **characterized in that** the at least silyl moiety is selected from -Si(CH₃)₃, -Si(C₂H₅)₃, -Si(CH₃)₂(C₄H₉), -Si(C₃H₇)₃, -Si(C₆H₅)₂(C₄H₉), mostly preferred -Si(C₃H₇)₃.

3. Method according to one of the preceding claims, **characterized in that** the at least one HF scavenger is K₂CO₃, Na₂CO₃, Cs₂CO₃.

4. Method according to one of the preceding claims, **characterized in that** the at least one organic solvent is selected from a group containing dichloromethane (DCM), Acetone, acetonitrile (MeCN), Diethyl ether, Nitromethane, Tetrahydrofuran (THF), Dimethyl sulphoxide (DMSO) and Ethyl acetate

5. Method according to one of the preceding claims, **characterized in that** the reaction is carried out at a temperature between 18°C and 25°C, preferably between 20°C and 23°C, such as room temperature.

6. Method according to one of the preceding claims, **characterized in that** reacting the organic compound with structural element (I) 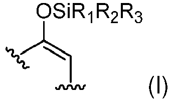 with Bis(trifluoromethyl)-peroxide (BTMP) in the presence of at least one HF scavenger and at least one organic solvent provides an organic compound comprising at least of the structural elements (III) or (IV) with one trifluoromethoxy moiety

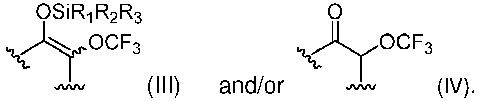

7. Method according to one of claims 6, **characterized in that** the organic compound with structural element (I) is one of the following compounds: 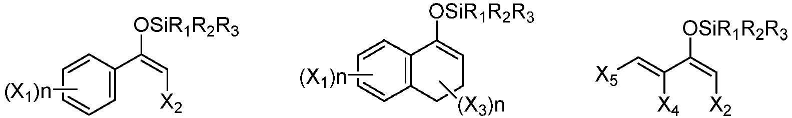 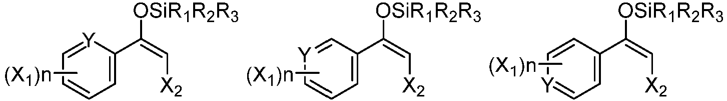 With
X₁ being H, alkyl, in particular C₁-C₆ alkyl, alkoxy, in particular C₁-C₆ alkoxy, -CN, halogen, in particular F, Cl, Br, I, -OCH₂C₆H₅ (OBn), -CH₂C₆H₅ (Bn), -CₙF₂ₙ₊₁, in particular -CF₃, -C₂F₅, an alkyl ring fused with C₆ aryl;
X₂ being H, alkyl, in particular C₁-C₆ alkyl, -CH₂C₆H₅ (Bn)
X₃ being H or alkyl, in particular C₁-C₆ alkyl,
X₄ being H or alkyl, in particular C₁-C₆ alkyl,
X₅ being alkyl, in particular C₁-C₆ alkyl, or cycloalkyl, in particular C₃-C₆ cycloalkyl, such as C₆ cycloalkyl,
n being 0-5, in particular 0, 1, 2 or 3, and
Y being N, S, or O.

8. Method according to claim 6 or 7, **characterized in that** reacting the organic compound with structural element (I) 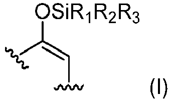 with Bis(trifluoromethyl)-peroxide (BTMP) in the presence of at least one HF scavenger and dichloromethane (DCM), diethyl ether and/or nitromethane, preferably dichloromethane (DCM), as the at least one organic solvent provides an organic compound comprising the structural element (III) with at least one trifluoromethoxy moiety

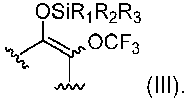

9. Method according to claim 6, **characterized in that** reacting the organic compound with structural element (I) 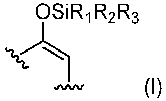 with Bis(trifluoromethyl)-peroxide (BTMP) in the presence of at least one HF scavenger and acetone or acetonitrile, preferably acetone, as the at least one organic solvent provides an organic compound comprising the structural element (IV) with at least one trifluoromethoxy moiety

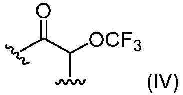

10. Method according to one of the claims 1-5, **characterized in that** reacting the organic compound with structural element (II) 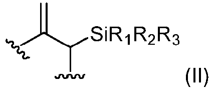 with Bis(trifluoromethyl)-peroxide (BTMP) in the presence of at least one HF scavenger and at least one organic solvent, preferably MeCN, provides an organic compound comprising the structural elements (V) with at least one trifluoromethoxy moiety

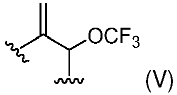

11. Method according to claim 10, **characterized in that** the organic compound with structural element (II) is one of the following compounds: 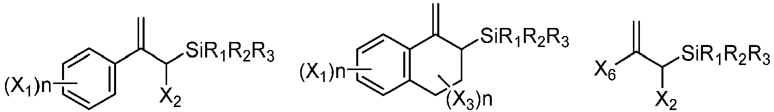 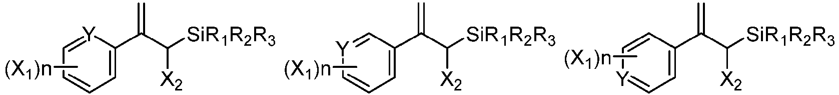 With
X₁ being H, alkyl, in particular C₁-C₆ alkyl, alkoxy, in particular C₁-C₆ alkoxy, Halogen, in particular F, Cl, Br, I, -OCH₂C₆H₅ or -CₙF₂ₙ₊₁, in particular -CF₃, -C₂F₅, CN, an alkyl ring fused with the C₆ aryl ,
X₂ being H, or alkyl, in particular C₁-C₆ alkyl,
X₃ being H, or alkyl, in particular C₁-C₆ alkyl,
X₆ being H, or alkyl, in particular C₁-C₃ alkyl,
n being 0-5, in particular 0, 1, 2, or 3, and
Y being, N, S, or O.

## Patentansprüche

1. Syntheseverfahren, vorzugsweise katalysatorfreies Syntheseverfahren, zum Einführen einer Trifluormethoxyeinheit in mindestens eine organische Verbindung, mit den folgenden Schritten:
- Bereitstellen mindestens einer organischen Verbindung, die mindestens eine Silyleinheit als Bestandteil der folgenden Strukturelemente (I) oder (II) umfasst 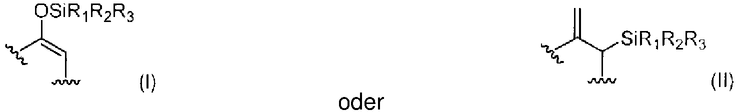 wobei R₁, R₂, R₃ aus C₁-C₄-Alkyl oder C₆-Aryl, insbesondere aus -CH₃, -C₂H₅, -C₃H₇, -C₄H₉, -C₆H₅ ausgewählt sind,
wobei R₁, R₂, R₃ gleich oder verschieden sein können,
- Umsetzen der mindestens einen organischen Verbindung mit den Strukturelementen (I) oder (II) mit Bis(trifluormethyl)-peroxid (BTMP) in Gegenwart von mindestens einem HF-Fänger und mindestens einem organischen Lösungsmittel, und
- Gewinnen mindestens einer organischen Verbindung, die mindestens eines der folgenden Strukturelemente (III), (IV) oder (V) mit mindestens einer Trifluormethoxyeinheit umfasst

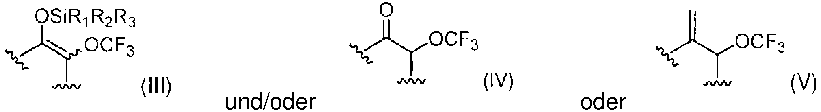

2. Verfahren nach Anspruch 1, **dadurch gekennzeichnet, dass** die mindestens eine Silyleinheit ausgewählt ist aus -Si(CH₃)₃, -Si(C₂H₅)₃, -Si(CH₃)₂(C₄H₉), -Si(C₃H₇)₃, -Si(C₆H₅)₂(C₄H₉), am meisten bevorzugt -Si(C₃H₇)₃.

3. Verfahren nach einem der vorhergehenden Ansprüche, **dadurch gekennzeichnet, dass** der mindestens eine HF-Fänger K₂CO₃, Na₂CO₃, Cs₂CO₃ ist.

4. Verfahren nach einem der vorhergehenden Ansprüche, **dadurch gekennzeichnet, dass** das mindestens eine organische Lösungsmittel aus einer Gruppe ausgewählt ist, die Dichlormethan (DCM), Aceton, Acetonitril (MeCN), Diethylether, Nitromethan, Tetrahydrofuran (THF), Dimethylsulfoxid (DMSO) und Ethylacetat enthält

5. Verfahren nach einem der vorhergehenden Ansprüche, **dadurch gekennzeichnet, dass** die Umsetzung bei einer Temperatur zwischen 18°C und 25°C, vorzugsweise zwischen 20°C und 23°C, beispielsweise bei Raumtemperatur, durchgeführt wird.

6. Verfahren nach einem der vorhergehenden Ansprüche, **dadurch gekennzeichnet, dass** die Umsetzung der organischen Verbindung mit dem Strukturelement (I) 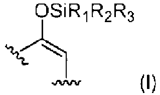 mit Bis(trifluormethyl)-peroxid (BTMP) in Gegenwart von mindestens einem HF-Fänger und mindestens einem organischen Lösungsmittel eine organische Verbindung liefert, die mindestens eines der Strukturelemente (III) oder (IV) mit einer Trifluormethoxyeinheit umfasst

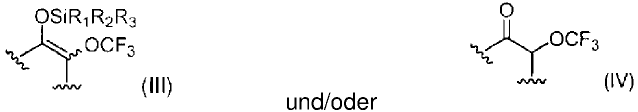

7. Verfahren nach Anspruch 6, **dadurch gekennzeichnet, dass** die organische Verbindung mit dem Strukturelement (I) eine der folgenden Verbindungen ist: 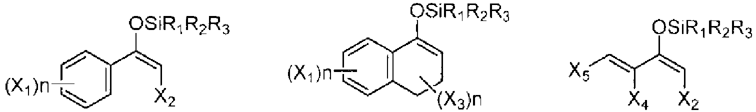 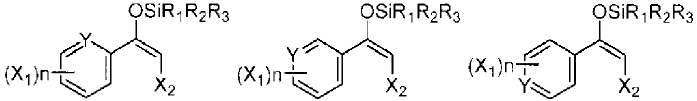 wobei
X₁ H, Alkyl, insbesondere C₁-C₆-Alkyl, Alkoxy ist, insbesondere C₁-C₆-Alkoxy, -CN, Halogen, insbesondere F, Cl, Br, I, -OCH₂C₆H₅ (OBn), -CH₂C₆H₅ (Bn), -CₙF₂ₙ₊₁, insbesondere -CF₃, -C₂F₅, ein mit C₆-Aryl kondensierter Alkylring;
X₂ H, Alkyl, insbesondere C₁-C₆ -Alkyl, -CH₂C₆H₅ (Bn) ist,
X₃ H oder Alkyl, insbesondere C₁-C₆-Alkyl ist,
X₄ H oder Alkyl, insbesondere C₁-C₆-Alkyl ist,
X₅ Alkyl, insbesondere C₁-C₆-Alkyl, oder Cycloalkyl ist, insbesondere C₃-C₆-Cycloalkyl, wie zum Beispiel C₆-Cycloalkyl,
n 0 bis 5 beträgt, insbesondere 0, 1, 2 oder 3, und
Y für N, S oder O steht.

8. Verfahren nach Anspruch 6 oder 7, **dadurch gekennzeichnet, dass** die Umsetzung der organischen Verbindung mit dem Strukturelement (I), 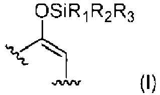 mit Bis(trifluormethyl)-peroxid (BTMP) in Gegenwart von mindestens einem HF-Fänger und Dichlormethan (DCM), Diethylether und/oder Nitromethan, vorzugsweise Dichlormethan (DCM), als dem mindestens einen organischen Lösungsmittel eine organische Verbindung liefert, die das Strukturelement (III) mit mindestens einer Trifluormethoxyeinheit umfasst

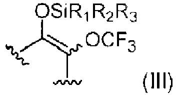

9. Verfahren nach Anspruch 6, **dadurch gekennzeichnet, dass** die Umsetzung der organischen Verbindung mit dem Strukturelement (I) 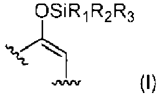 mit Bis(trifluormethyl)-peroxid (BTMP) in Gegenwart von mindestens einem HF-Fänger und Aceton oder Acetonitril, vorzugsweise Aceton, als dem mindestens einen organischen Lösungsmittel eine organische Verbindung liefert, die das Strukturelement (IV) mit mindestens einer Trifluormethoxyeinheit umfasst

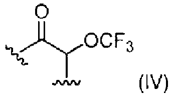

10. Verfahren nach einem der Ansprüche 1-5, **dadurch gekennzeichnet, dass** die Umsetzung der organischen Verbindung mit dem Strukturelement (II) 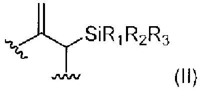 mit Bis(trifluormethyl)-peroxid (BTMP) in Gegenwart von mindestens einem HF-Fänger und mindestens einem organischen Lösungsmittel, vorzugsweise MeCN, eine organische Verbindung liefert, die die Strukturelemente (V) mit mindestens einer Trifluormethoxyeinheit umfasst

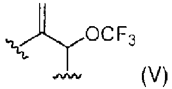

11. Verfahren nach Anspruch 10, **dadurch gekennzeichnet, dass** die organische Verbindung mit dem Strukturelement (II) eine der folgenden Verbindungen ist: 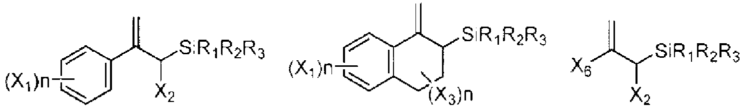 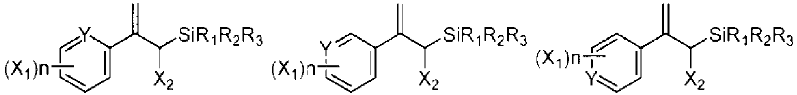 wobei
X₁ H, Alkyl, insbesondere C₁-C₆-Alkyl, Alkoxy ist, insbesondere C₁-C₆-Alkoxy, Halogen, insbesondere F, Cl, Br, I, -OCH₂C₆H₅ oder -CₙF₂ₙ₊₁, insbesondere -CF₃, -C₂F₅, CN, ein mit C₆-Aryl kondensierter Alkylring,
X₂ H oder Alkyl, insbesondere C₁-C₆-Alkyl ist,
X₃ H oder Alkyl, insbesondere C₁-C₆-Alkyl ist,
X₆ H oder Alkyl, insbesondere C₁-C₈-Alkyl ist,
n 0 bis 5 beträgt, insbesondere 0, 1, 2 oder 3, und
Y für N, S oder O steht.

## Revendications

1. Procédé de synthèse, de préférence un procédé de synthèse sans catalyseur, pour introduire un fragment trifluorométhoxy dans au moins un composé organique, comprenant les étapes suivantes :
- fournir au moins un composé organique comprenant au moins un fragment silyle faisant partie des éléments structuraux (I) ou (II) suivants 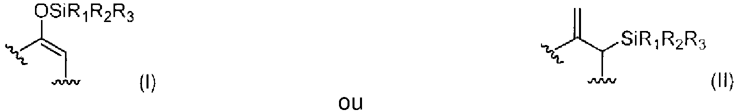 R₁, R₂ et R₃ étant choisis parmi les groupes alkyle en C₁-C₄ ou aryle en C₆, en particulier -CH₃, -C₂H₅, -C₃H₇, -C₄H₉, -C₆H₅,
dans lequel R₁, R₂, R₃ peuvent être identiques ou différents les uns des autres,
- faire réagir l'au moins un composé organique avec les éléments structuraux (I) ou (II) avec du peroxyde de bis(trifluorométhyle) (BTMP) en présence d'au moins un agent piégeur de HF et d'au moins un solvant organique, et
- obténir au moins un composé organique comprenant au moins l'un des éléments structuraux suivants (III), (IV) ou (V) avec au moins un fragment trifluorométhoxy

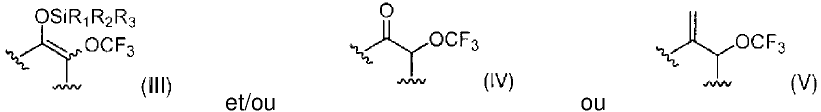

2. Procédé selon la revendication 1, **caractérisé en ce que** l'au moins un fragment silyle est choisi parmi -Si(CH₃)₃, -Si(C₂H₅)₃, -Si(CH₃)₂(C₄H₉), -Si(C₃H₇)₃, -Si(C₆H₅)₂(C₄H₉), de préférence -Si(C₃H₇)₃.

3. Procédé selon l'une quelconque des revendications précédentes, **caractérisé en ce que** l'au moins un agent piégeur de HF est K₂CO₃, Na₂CO₃, Cs₂CO₃.

4. Procédé selon l'une quelconque des revendications précédentes, **caractérisé en ce que** ledit au moins un solvant organique est choisi parmi un groupe comprenant le dichlorométhane (DCM), l'acétone, l'acétonitrile (MeCN), l'éther diéthylique, le nitrométhane, le tétrahydrofurane (THF), le diméthylsulfoxyde (DMSO) et l'acétate d'éthyle

5. Procédé selon l'une quelconque des revendications précédentes, **caractérisé en ce que** la réaction est effectuée à une température comprise entre 18 °C et 25 °C, de préférence entre 20 °C et 23 °C, telle que la température ambiante.

6. Procédé selon l'une quelconque des revendications précédentes, **caractérisé en ce que** faire réagir le composé organique comportant l'élément structural (I) 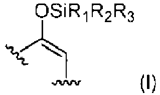 avec du peroxyde de bis(trifluorométhyle) (BTMP) en présence d'au moins un agent piégeur de HF et d'au moins un solvant organique fournit un composé organique comprenant au moins l'un des éléments structuraux (III) ou (IV) comportant un fragment trifluorométhoxy

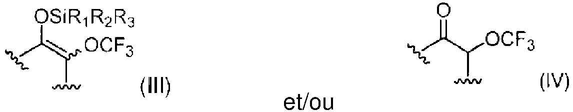

7. Procédé selon la revendication 6, **caractérisé en ce que** le composé organique comportant l'élément structural (I) est l'un des composés suivants : 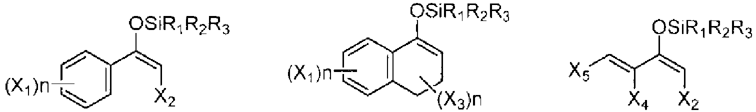 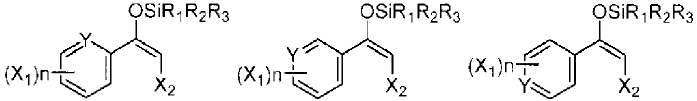 où
X₁ représente H, alkyle, en particulier alkyle en C₁-C₆, alcoxy, en particulier alcoxy en C₁-C₆, -CN, halogène, en particulier F, Cl, Br, I, -OCH₂C₆H₅ (OBn), -CH₂C₆H₅ (Bn), -CₙF₂ₙ₊₁, en particulier -CF₃, -C₂F₅, un cycle alkyle condensé avec un groupe aryle en C₆;
X₂ représente H, alkyle, en particulier alkyle en C₁-C₆, -CH₂C₆H₅ (Bn),
X₃ représente H ou alkyle, en particulier alkyle en C₁-C₆,
X₄ représente H ou alkyle, en particulier alkyle en C₁-C₆,
X₅ représente alkyle, en particulier alkyle en C₁-C₆, ou cycloalkyle, en particulier cycloalkyle en C₃-C₆, tel que cycloalkyle en C₆,
n représente 0-5, en particulier 0, 1, 2, ou 3, et
Y représente N, S ou O.

8. Procédé selon la revendication 6 ou 7, **caractérisé en ce que** faire réagir le composé organique comportant l'élément structural (I) 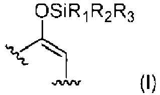 avec du peroxyde de bis(trifluorométhyle) (BTMP) en présence d'au moins un agent piégeur de HF et de dichlorométhane (DCM), d'éther diéthylique et/ou de nitrométhane, de préférence de dichlorométhane (DCM), en tant qu'au moins un solvant organique, fournit un composé organique comprenant l'élément structural (III) avec au moins un fragment trifluorométhoxy

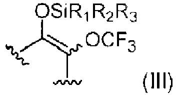

9. Procédé selon la revendication 6, **caractérisé en ce que** faire réagir le composé organique comportant l'élément structural (I) 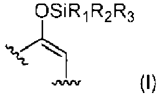 avec du peroxyde de bis(trifluorométhyle) (BTMP) en présence d'au moins un agent piégeur de HF et d'acétone ou d'acétonitrile, de préférence d'acétone, en tant qu'au moins un solvant organique, fournit un composé organique comprenant l'élément structural (IV) avec au moins un fragment trifluorométhoxy

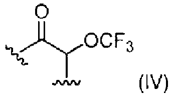

10. Procédé selon l'une quelconque des revendications 1-5, **caractérisé en ce que** faire réagir le composé organique comportant l'élément structural (II) 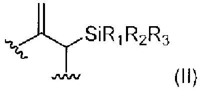 avec du peroxyde de bis(trifluorométhyle) (BTMP) en présence d'au moins un agent piégeur de HF et d'au moins un solvant organique, de préférence du MeCN, fournit un composé organique comprenant les éléments structuraux (V) comportant au moins un fragment trifluorométhoxy

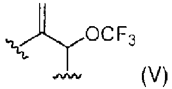

11. Procédé selon la revendication 10, **caractérisé en ce que** le composé organique comportant l'élément structural (II) est l'un des composés suivants : 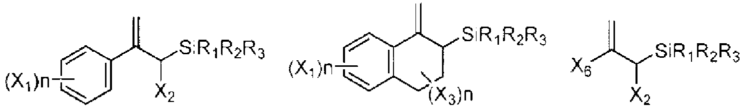 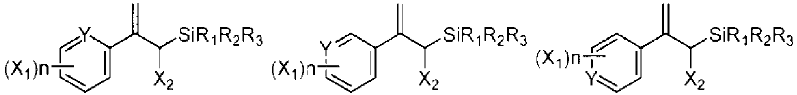 où
X₁ représente H, alkyle, en particulier alkyle en C₁-C₆, alcoxy, en particulier alcoxy en C₁-C₆, halogène, en particulier F, Cl, Br, I, -OCH₂C₆H₅ ou -CₙF₂ₙ₊₁, en particulier -CF₃, -C₂F₅, CN, un cycle alkyle condensé avec un groupe aryle en C₆,
X₂ représente H ou alkyle, en particulier alkyle en C₁-C₆,
X₃ représente H ou alkyle, en particulier alkyle en C₁-C₆,
X₆ représente H ou alkyle, en particulier alkyle en C₁-C₈, n représente 0-5, en particulier 0, 1, 2, ou 3, et
Y représente N, S ou O.
